# EUROPEAN PATENT APPLICATION

(11) **EP 4 019 497 A1**
(43) Date of publication of application: **29.06.2022**
(21) Application number: 20854797.6
(22) Date of filing: 14.08.2020
(51) Int. Cl.: C07D 209/48

(54) **PRODUCTION METHOD FOR NITROGEN-CONTAINING FLUORINE-CONTAINING COMPOUND**

(30) Priority: 20.08.2019 JP 2019150741
(71) Applicant: AGC INC., Chiyoda-ku, Tokyo 1008405 (JP)
(72) Inventor: OKAZOE Takashi, Tokyo 100-8405 (JP); ISHIBASHI Yuichiro, Tokyo 100-8405 (JP)
(74) Representative: Müller-Boré & Partner Patentanwälte PartG mbB
(86) International application number: PCT/JP2020/030908
(87) International publication number: WO 2021/033649

(57) **Abstract**

The present invention pertains to a nitrogen-containing fluorine-containing compound production method that is characterized by including a step in which a compound indicated by formula (5) is fluorinated and a compound indicated by formula (6) is obtained. R3: A single bond or C1-20 divalent organic group. R4 and R5: At least one is a monovalent organic group having an electron withdrawing group. R6: A C1-20 monovalent organic group. E3: A divalent organic group. R3F, R4F, R5F, R6F, and E3F: Groups having some or all hydrogen atoms in R3, R4, R5, R6, and E3 fluorinated.

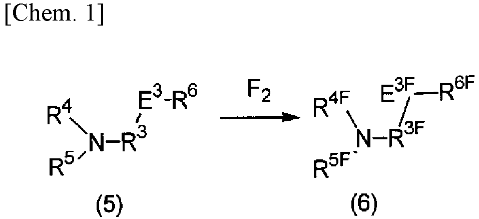

## Description

### TECHNICAL FIELD

The present invention relates to a method for producing a nitrogen-containing fluorine-containing compound. The present invention further relates to novel nitrogen-containing fluorine-containing compounds.

### BACKGROUND ART

Nitrogen-containing fluorine-containing compounds include compounds which are industrially useful as solvents, etc., and production methods therefor have hence been developed hitherto. For example, known as methods for fluorinating all the C-H moieties of a C-H-containing compound into C-F are a method in which cobalt trifluoride is used (Patent Document 1), a method in which hydrogen fluoride is electrolyzed in an electrolysis vessel to conduct a fluorination reaction (hereinafter referred to as ECF method) (Patent Document 2), a method in which fluorine gas is used to directly conduct a fluorination reaction in a liquid phase (Patent Document 3), etc.

### CITATION LIST

### PATENT LITERATURE

Patent Document 1: JP-T-S60-500498 (The term "JP-T" as used herein means a published Japanese translation of a PCT patent application.)
Patent Document 2: British Patent No. 718318
Patent Document 3: International Publication WO 2002/026693

### SUMMARY OF INVENTION

### TECHNICAL PROBLEMS

However, the method described in Patent Document 1 has a problem in that since a gas-solid reaction is performed at a high temperature, isomerization and linkage scission occur to yield by-products of many kinds.

The method described in Patent Document 2 has a problem in that the fluorination is accompanied with isomerization and the isomers are difficult to separate and, hence, a fluorinated trialkylamine is yielded as an isomer mixture.

Patent Document 3 describes a feature wherein a hydrogenfluoride of R₂N-R-E-R is formed and then fluorinated. The hydrogenfluoride has low solubility in the solvent for use in the fluorination reaction (in Patent Document 3, the solvent is referred to as "solvent 2") and it is necessary to use the solvent in a large amount for conducting the reaction in an even solution, making the method industrially disadvantageous. If the amount of the solvent is reduced in order to heighten the volumetric efficiency, the solid starting material reacts with the fluorine gas, resulting in a problem in that the heat removal becomes insufficient and the scission of C-C single bonds occurs during the fluorination reaction to yield by-products of many kinds.

In the conventional methods, there has hence been room for improvement in yield in the fluorination reactions, because of by-products, etc.

An object of the present invention is to provide, in view of such problems of the prior-art techniques, a method for producing a nitrogen-containing fluorine-containing compound, the method attaining a high yield in a fluorination reaction.

### SOLUTION TO PROBLEMS

The present invention includes the following configurations.
[1] A method for producing a nitrogen-containing fluorine-containing compound, the method including a step in which a compound represented by the following formula (5) is fluorinated to obtain a compound represented by the following formula (6).

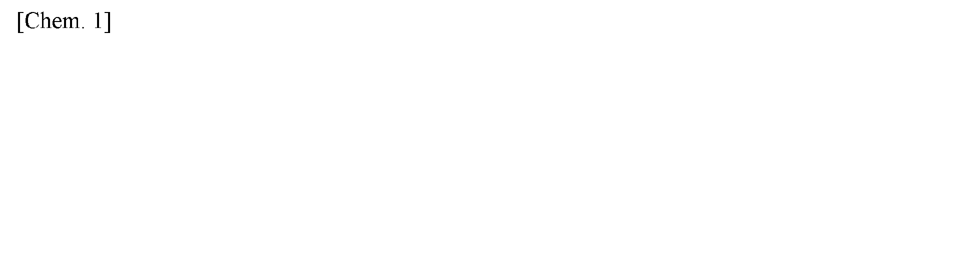

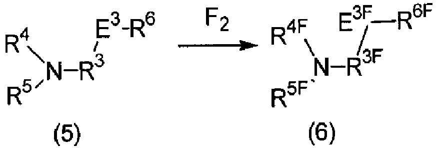

Definitions of substituents in the formulae are as follows.

R³: a single bond or a divalent organic group having 1-20 carbon atoms.

R⁴ and R⁵ are each independently a monovalent organic group having an electron-withdrawing group, a monovalent hydrocarbon group having 1-20 carbon atoms, or a monovalent hydrocarbon group having 2-20 carbon atoms which contains an etheric oxygen atom between carbon atoms, at least one of R⁴ and R⁵ being a monovalent organic group having an electron-withdrawing group. R³, R⁴, and R⁵ may be bonded to each other to form a divalent organic group or a trivalent organic group.

R⁶: a monovalent organic group having 1-20 carbon atoms.

E³: a divalent organic group formed by a reaction between the following E¹ and E².

E¹: -COX, -SO₂X, or -C(R^{E11})(R^{E12})OH. X is a hydroxyl group or a halogen atom. R^{E11} and R^{E12} are each independently a hydrogen atom or a monovalent organic group having 1-10 carbon atoms.

E²: -C(R^{E21})(R^{E22})OH, -COX, or -SO₂X. R^{E21} and R^{E22} are each independently a hydrogen atom or a monovalent organic group having 1-20 carbon atoms. X is a hydroxyl group or a halogen atom.

R^{3F}: a group formed by fluorinating some or all of hydrogen atoms of R³.

R^{4F}: a group formed by fluorinating some or all of hydrogen atoms of R⁴.

R^{5F}: a group formed by fluorinating some or all of hydrogen atoms of R⁵.

R^{6F}: a group formed by fluorinating some or all of hydrogen atoms of R⁶.

E^{3F}: a group formed by fluorinating some or all of hydrogen atoms of E³.

[2] The production method according to [1] which further includes a step in which a compound represented by the following formula (3)-1 is reacted with a compound represented by the following formula (4) to obtain the compound represented by the following formula (5) or
a step in which a compound represented by the following formula (3)-2 is reacted with a compound having an amino-protecting group to obtain the compound represented by the following formula (5).

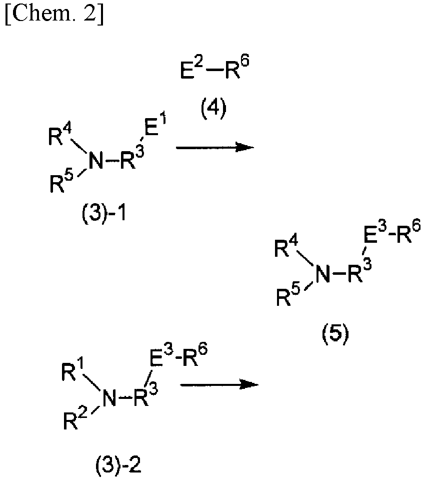

Definitions of substituents in the formulae are as follows.

R¹ and R² are each independently a hydrogen atom, a monovalent hydrocarbon group having 1-20 carbon atoms, or a monovalent hydrocarbon group having 2-20 carbon atoms which contains an etheric oxygen atom between carbon atoms, at least one of R¹ and R² being a hydrogen atom. In the case where R¹ or R² is not a hydrogen atom, then the R¹ or R² may be bonded to R³ to form a divalent organic group.

R³, R⁴, R⁵, R⁶, E¹, E², and E³: the same as in [1].

[3] The production method according to [2] which further includes a step in which a compound represented by the following formula (1) is reacted with a compound having an amino-protecting group to obtain the compound represented by the following formula (3)-1 or
a step in which a compound represented by the following formula (1) is reacted with a compound represented by the following formula (4) to obtain the compound represented by the following formula (3)-2.

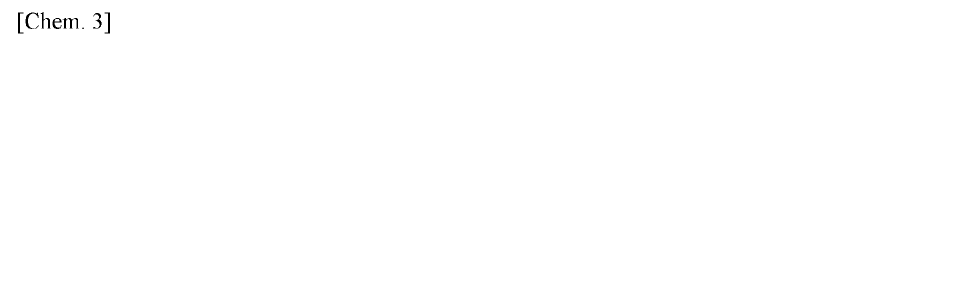

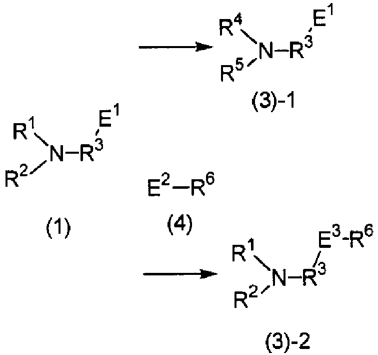

Definitions of substituents in the formulae are as follows.

R¹, R², R³, R⁴, R⁵, R⁶, E¹, E², and E³: the same as in [1] or [2].

[4] The production method according to any one of [1] to [3] which further includes a step in which the compound represented by the following formula (6) is deprotected and caused to undergo scission of the linking group E^{3F}, thereby obtaining a compound represented by the following formula (7).

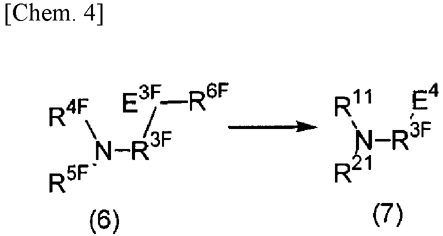

Definitions of substituents in the formulae are as follows.

R¹¹ and R²¹ are each independently a hydrogen atom or a monovalent organic group having 1-20 carbon atoms, at least one of R¹¹ and R²¹ being a hydrogen atom. In the case where R¹¹ or R²¹ is not a hydrogen atom, then the R¹¹ or R²¹ may be bonded to R^{3F} to form a divalent organic group.

E⁴: -COX or -SO₂X. X is a hydroxyl group or a halogen atom.

R^{3F}, R^{4F}, R^{5F}, R^{6F}, and E^{3F}: the same as in [1].

[5] A compound represented by the following formula (5).

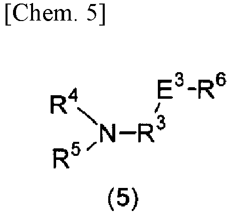

Definitions of substituents in the formula are as follows.

R³: a single bond or a divalent organic group having 1-20 carbon atoms.

R⁴ and R⁵ are each independently a monovalent organic group having an electron-withdrawing group, a monovalent hydrocarbon group having 1-20 carbon atoms, or a monovalent hydrocarbon group having 2-20 carbon atoms which contains an etheric oxygen atom between carbon atoms, at least one of R⁴ and R⁵ being a monovalent organic group having an electron-withdrawing group. R³, R⁴, and R⁵ may be bonded to each other to form a divalent organic group or a trivalent organic group.

R⁶: a monovalent organic group having 1-20 carbon atoms.

E³: a divalent organic group formed by a reaction between the following E¹ and E².

E¹: -COX, -SO₂X, or -C(R^{E11})(R^{E12})OH. X is a hydroxyl group or a halogen atom. R^{E11} and R^{E12} are each independently a hydrogen atom or a monovalent organic group having 1-10 carbon atoms.

E²: -C(R^{E21})(R^{E22})OH, -COX, or -SO₂X. R^{E21} and R^{E22} are each independently a hydrogen atom or a monovalent organic group having 1-20 carbon atoms. X is a hydroxyl group or a halogen atom.

[6] A compound represented by the following formula (5a) or a compound represented by the following formula (5b).

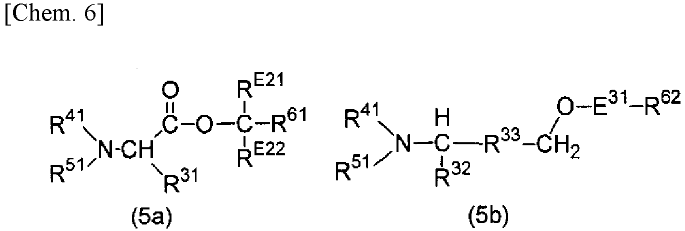

Definitions of substituents in the formulae are as follows.

R³¹: a hydrogen atom or a monovalent organic group having 1-19 carbon atoms.

R³²: a hydrogen atom or a monovalent organic group having 1-19 carbon atoms.

R³³: a single bond or a divalent organic group having 1-10 carbon atoms.

R⁴¹: a monovalent organic group having an electron-withdrawing group, a monovalent hydrocarbon group having 1-20 carbon atoms, or a monovalent hydrocarbon group having 2-20 carbon atoms which contains an etheric oxygen atom between carbon atoms.

R⁵¹: a monovalent organic group having an electron-withdrawing group. R³¹, R⁴¹, and R⁵¹ may be bonded to each other to form a divalent organic group or a trivalent organic group. R³², R⁴¹, and R⁵¹ may be bonded to each other to form a divalent organic group or a trivalent organic group.

R^{E21} and R^{E22}: each independently a hydrogen atom or a monovalent organic group having 1-20 carbon atoms.

R⁶¹: a monovalent organic group having 1-20 carbon atoms.

R⁶²: a monovalent organic group having 1-20 carbon atoms.

E³¹: -CO- or -SO₂-

[7] A compound represented by the following formula (6).

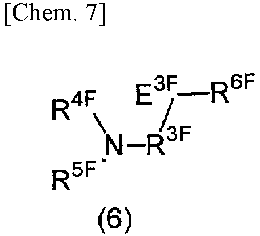

Definitions of substituents in the formula are as follows.

R^{3F}: a single bond or a group formed by fluorinating some or all of hydrogen atoms of a divalent organic group having 1-20 carbon atoms.

R^{4F}: a group formed by fluorinating some or all of hydrogen atoms of the following R⁴_{.}

R^{5F}: a group formed by fluorinating some or all of hydrogen atoms of the following R⁵.

R⁴ and R⁵ are each independently a monovalent organic group having an electron-withdrawing group, a monovalent hydrocarbon group having 1-20 carbon atoms, or a monovalent hydrocarbon group having 2-20 carbon atoms which contains an etheric oxygen atom between carbon atoms, at least one of R⁴ and R⁵ being a monovalent organic group having an electron-withdrawing group. R^{3F}, R^{4F}, and R^{5F} may be bonded to each other to form a divalent organic group or a trivalent organic group.

R^{6F}: a group formed by fluorinating some or all of hydrogen atoms of a monovalent organic group having 1-20 carbon atoms.

E^{3F}: a group formed by fluorinating some or all of hydrogen atoms of a divalent organic group formed by a reaction between the following E¹ and E².

E¹: -COX, -SO₂X, or -C(R^{E11})(R^{E12})OH. X is a hydroxyl group or a halogen atom. R^{E11} and R^{E12} are each independently a hydrogen atom or a monovalent organic group having 1-10 carbon atoms.

E²: -C(R^{E21})(R^{E22})OH, -COX, or -SO₂X. R^{E21} and R^{E22} are each independently a hydrogen atom or a monovalent organic group having 1-20 carbon atoms. X is a hydroxyl group or a halogen atom.

[8] A compound represented by the following formula (6a)-1, a compound represented by the following formula (6a)-2, a compound represented by the following formula (6b)-1, or a compound represented by the following formula (6b)-2.

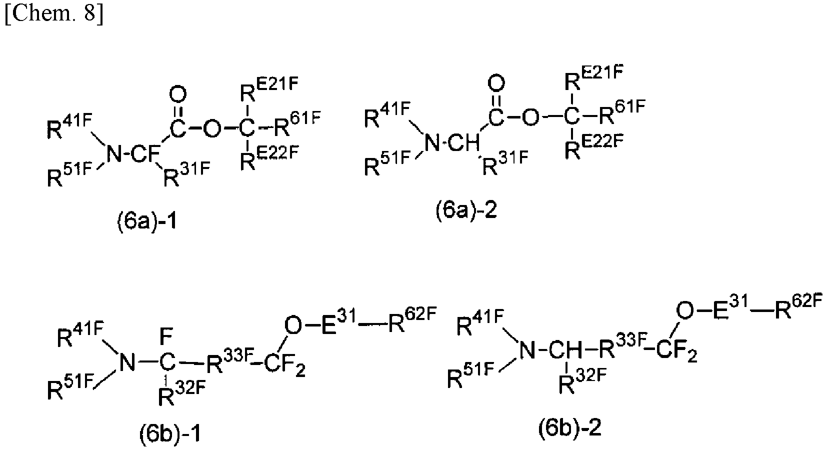

Definitions of substituents in the formulae are as follows.

R^{31F}: a fluorine atom or a group formed by fluorinating some or all of hydrogen atoms of a monovalent organic group having 1-19 carbon atoms.

R^{32F}: a fluorine atom or a group formed by fluorinating some or all of hydrogen atoms of a monovalent organic group having 1-19 carbon atoms.

R^{33F}: a single bond or a group formed by fluorinating some or all of hydrogen atoms of a divalent organic group having 1-10 carbon atoms.

R^{41F}: a group formed by fluorinating some or all of hydrogen atoms of the following R⁴¹.

R^{51F}: a group formed by fluorinating some or all of hydrogen atoms of the following R⁵¹.

R⁴¹: a monovalent organic group having an electron-withdrawing group, a monovalent hydrocarbon group having 1-20 carbon atoms, or a monovalent hydrocarbon group having 2-20 carbon atoms which contains an etheric oxygen atom between carbon atoms.

R⁵¹: a monovalent organic group having an electron-withdrawing group. R^{31F}, R^{41F}, and R^{51F} may be bonded to each other to form a divalent organic group or a trivalent organic group. R^{32F}, R^{41F}, and R^{51F} may be bonded to each other to form a divalent organic group or a trivalent organic group.

R^{E21F} and R^{E22F}: each independently a fluorine atom or a group formed by fluorinating some or all of hydrogen atoms of a monovalent organic group having 1-20 carbon atoms.

R^{61F} and R^{62F}: each independently a group formed by fluorinating some or all of hydrogen atoms of a monovalent organic group having 1-20 carbon atoms.

E³¹: -CO- or -SO₂-.

[9] A method for producing a nitrogen-containing fluorine-containing compound, the method including a step in which a compound represented by the following formula (1a) is reacted with a compound having an amino-protecting group to obtain a compound represented by the following formula (3a)-1 and
a step in which the compound represented by the following formula (3a)-1 is reacted with a compound represented by the following formula (4a) to obtain a compound represented by the following formula (5a)
or including a step in which a compound represented by the following formula (1a) is reacted with a compound represented by the following formula (4a) to obtain a compound represented by the following formula (3a)-2 and
a step in which the compound represented by the following formula (3a)-2 is reacted with a compound having an amino-protecting group to obtain a compound represented by the following formula (5a),
the method further including a step in which the compound represented by the following formula (5a) is fluorinated to obtain at least one of a compound represented by the following formula (6a)-1 and a compound represented by the following formula (6a)-2.

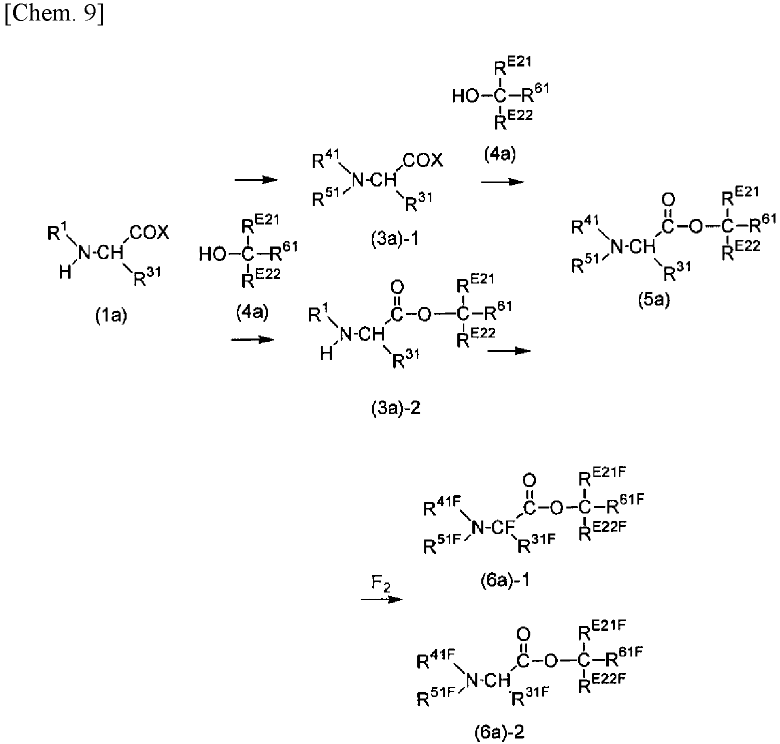

Definitions of substituents in the formulae are as follows.

R¹ is a hydrogen atom, a monovalent hydrocarbon group having 1-20 carbon atoms, or a monovalent hydrocarbon group having 2-20 carbon atoms which contains an etheric oxygen atom between carbon atoms. In the case where R¹ is not a hydrogen atom, the R¹ may be bonded to R³¹ to form a divalent organic group.

R³¹: a hydrogen atom or a monovalent organic group having 1-19 carbon atoms.

X: a hydroxyl group or a halogen atom.

R⁴¹: a monovalent organic group having an electron-withdrawing group, a monovalent hydrocarbon group having 1-20 carbon atoms, or a monovalent hydrocarbon group having 2-20 carbon atoms which contains an etheric oxygen atom between carbon atoms.

R⁵¹: a monovalent organic group having an electron-withdrawing group. R³¹, R⁴¹, and R⁵¹ may be bonded to each other to form a divalent organic group or a trivalent organic group.

R^{E21} and R^{E22}: each independently a hydrogen atom or a monovalent organic group having 1-20 carbon atoms.

R⁶¹: a monovalent organic group having 1-20 carbon atoms.

R^{41F}: a group formed by fluorinating some or all of hydrogen atoms of R⁴¹.

R^{51F}: a group formed by fluorinating some or all of hydrogen atoms of R⁵¹.

R^{61F}: a group formed by fluorinating some or all of hydrogen atoms of R⁶¹.

R^{E21F} and R^{E22F}: each independently a fluorine atom or a group formed by fluorinating some or all of hydrogen atoms of a monovalent organic group having 1-20 carbon atoms.

[10] A method for producing a nitrogen-containing fluorine-containing compound, the method including a step in which a compound represented by the following formula (1b) is reacted with a compound having an amino-protecting group to obtain a compound represented by the following formula (3b)-1 and
a step in which the compound represented by the following formula (3b)-1 is reacted with a compound represented by the following formula (4b) to obtain a compound represented by the following formula (5b)
or including a step in which a compound represented by the following formula (1b) is reacted with a compound represented by the following formula (4b) to obtain a compound represented by the following formula (3b)-2 and
a step in which the compound represented by the following formula (3b)-2 is reacted with a compound having an amino-protecting group to obtain a compound represented by the following formula (5b),
the method further including a step in which the compound represented by the following formula (5b) is fluorinated to obtain at least one of a compound represented by the following formula (6b)-1 and a compound represented by the following formula (6b)-2.

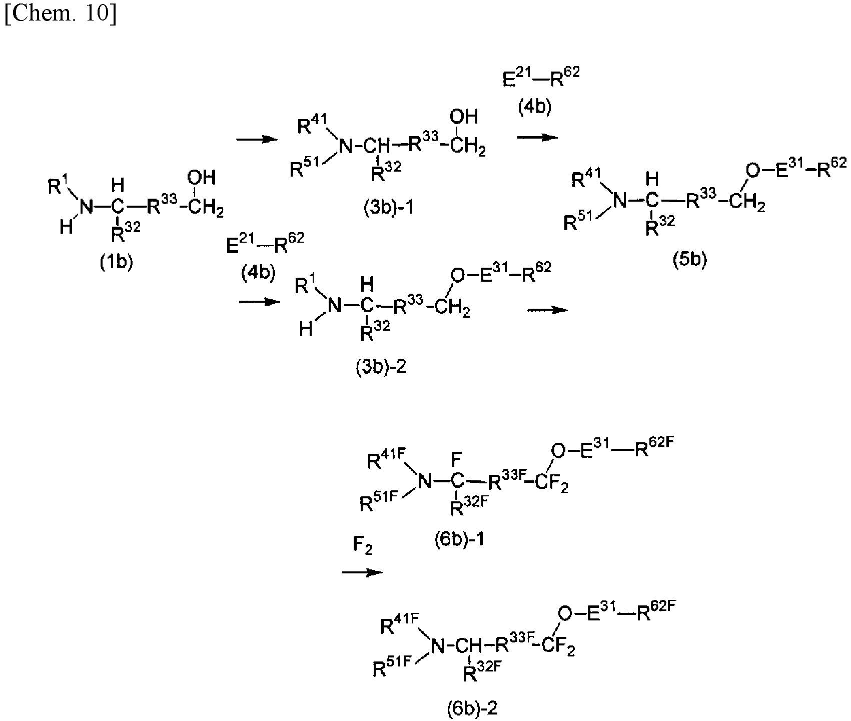

Definitions of substituents in the formulae are as follows.

R¹ is a hydrogen atom, a monovalent hydrocarbon group having 1-20 carbon atoms, or a monovalent hydrocarbon group having 2-20 carbon atoms which contains an etheric oxygen atom between carbon atoms. In the case where R¹ is not a hydrogen atom, the R¹ may be bonded to R³² to form a divalent organic group.

R³²: a hydrogen atom or a monovalent organic group having 1-19 carbon atoms.

R³³: a single bond or a divalent organic group having 1-10 carbon atoms, a total number of carbon atoms of R³² and R³³ being 0-19.

R⁴¹: a monovalent organic group having an electron-withdrawing group, a monovalent hydrocarbon group having 1-20 carbon atoms, or a monovalent hydrocarbon group having 2-20 carbon atoms which contains an etheric oxygen atom between carbon atoms.

R⁵¹: a monovalent organic group having an electron-withdrawing group. R³², R⁴¹, and R⁵¹ may be bonded to each other to form a divalent organic group or a trivalent organic group.

E²¹: -COX or -SO₂X. X is a hydroxyl group or a halogen atom.

R⁶²: a monovalent organic group having 1-20 carbon atoms.

E³¹: -CO- or -SO₂-.

R^{32F}: a group formed by fluorinating some or all of hydrogen atoms of R³².

R^{33F}: a group formed by fluorinating some or all of hydrogen atoms of R³³, a total number of carbon atoms of R^{32F} and R^{33F} being 0-19.

R^{41F}: a group formed by fluorinating some or all of hydrogen atoms of R⁴¹.

R^{51F}: a group formed by fluorinating some or all of hydrogen atoms of R⁵¹.

R^{62F}: a group formed by fluorinating some or all of hydrogen atoms of R⁶².

### ADVANTAGEOUS EFFECTS OF INVENTION

According to the present invention, the amino group of an amine compound used as a starting material is protected with a group including an electron-withdrawing group, thereby rendering HF to be generated as a by-product of fluorination reaction less apt to form a hydrogenfluoride with the amine compound. As a result, the fluorination reaction can be conducted while preventing the starting-material compounds from decreasing in solubility in the solvent. Thus, the fluorination reaction can be carried out with high yield.

### DESCRIPTION OF EMBODIMENTS

In this description, "compound represented by formula (1)" is referred to as "compound (1)".

In this description, the term "organic group" means a group including one or more carbon atoms as an essential component.

The term "fluorination" means replacing one or more hydrogen atoms each by a fluorine atom.

The term "hydrocarbon group" means any of a linear, branched, or cyclic, saturated aliphatic hydrocarbon group, a linear, branched, or cyclic, unsaturated aliphatic hydrocarbon group, and an aromatic hydrocarbon group.

### «Production of Compound (5)»

In the present invention, compound (5), which is an amine compound to be subjected to a fluorination reaction, may be a commercial product or can be produced, for example, by the following method.

Compound (1) is prepared first, and compound (2) and compound (4) are reacted, thereby obtaining compound (5).

Specifically, by reacting compound (1) with compound (2), the amino group of the compound (1) is protected. In the case where R¹ and/or R² in compound (1) is a hydrogen atom, this hydrogen atom is an active proton having high reactivity. By protecting the amino group before fluorination, an abrupt reaction with fluorine gas can be avoided in the fluorination to be performed later.

By reacting compound (1) with compound (4), some of the structure of the compound (4) is linked to the compound (1), resulting in an increase in molecular weight. In the case where a starting material is subjected to a fluorination reaction in a liquid phase using fluorine gas and when the starting material has a low boiling point, then the starting material is prone to undergo a decomposition reaction in the gas phase. Fluorination can be conducted while preventing such decomposition reaction, by selecting starting-material compound (1) which is available at low cost, converting the compound (1) into a compound having a specific structure which has a high molecular weight for making the compound less apt to undergo a gas-phase reaction and which renders the compound soluble in a solvent to be used in a fluorination reaction, and thereafter conducting fluorination in a liquid phase. Furthermore, the linking group is cut after the fluorination. Thus, the desired fluorinated amine compound can be produced.

In reacting compound (1) with compound (2) and compound (4), the order of reaction is not particularly limited. In the case where compound (1) is reacted first with compound (2), compound (3)-1 is yielded, and compound (4) is subsequently reacted to thereby obtain compound (5). In the case where compound (1) is reacted first with compound (4), compound (3)-2 is yielded, and compound (2) is subsequently reacted to thereby obtain compound (5).

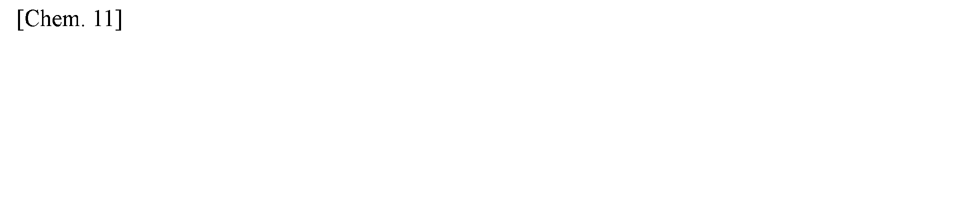

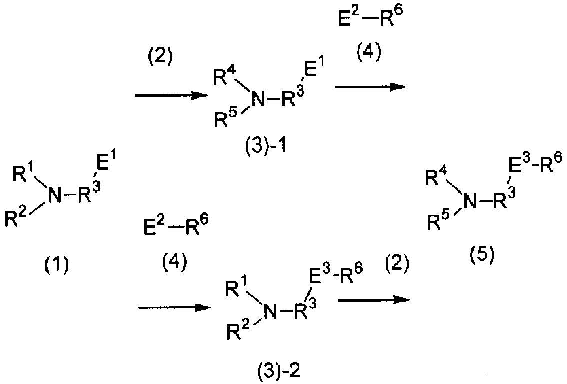

### <Compound (1)>

R¹ and R² are each independently a hydrogen atom, a monovalent hydrocarbon group having 1-20 carbon atoms, or a monovalent hydrocarbon group having 2-20 carbon atoms which contains an etheric oxygen atom between carbon atoms.

The monovalent hydrocarbon groups are preferably alkyl groups.

The number of carbon atoms of the monovalent hydrocarbon group having 1-20 carbon atoms is preferably 1-8, more preferably 1-4.

The number of carbon atoms of the monovalent hydrocarbon group having 2-20 carbon atoms which contains an etheric oxygen atom between carbon atoms is preferably 2-10. Examples thereof include CH₃(OCH₂CH₂)ₜ-(t=1-4). More preferred is CH₃(OCH₂CH₂)ₜ-(t=1-2).

At least one of R¹ and R² is a hydrogen atom.

When R¹ or R² is not a hydrogen atom, the R¹ or R² may be bonded to R³ to form a divalent organic group. Thus, a ring is formed. In this case, the divalent organic group formed by the bonding of the R¹ or R² to R³ is a divalent hydrocarbon group having 1-20 carbon atoms or a divalent hydrocarbon group having 2-20 carbon atoms which contains an etheric oxygen atom between carbon atoms.

R³ is a single bond or a divalent organic group having 1-20 carbon atoms.

R³ may be bonded to either of R⁴ and R⁵, which is described later, to form a ring.

When R³ is a single bond, E¹, which is described later, is preferably -C(R^{E11})(R^{E12})OH.

The divalent organic group is preferably a divalent hydrocarbon group. The number of carbon atoms of the divalent organic group is preferably 1-10. The divalent organic group may have a substituent.

The divalent organic group having 1-20 carbon atoms is preferably a divalent hydrocarbon group having 1-10 carbon atoms which may have a substituent. Examples of the substituent include a carboxyl group, an amino group, a benzene ring, a heterocycle, a hydroxyl group, a thiol group, a thioether group, an amide group, an imino group, and a guanidyl group. Preferred as the heterocycle are nitrogen-containing heterocycles, and especially preferred are a pyrrole ring, an imidazole ring, and an indole ring. From the standpoints of inhibiting an abrupt reaction with fluorine gas and improving the solubility in a solvent in the fluorination reaction, some or all of the hydrogen atoms of R³ may have been replaced by fluorine atoms. In the case where R³ has a substituent, the number of carbon atoms of the substituent is included in the number of carbon atoms of R³. In the case where the substituent has active hydrogen like a carboxyl, amino, hydroxyl, thiol, amide, or guanidyl group, it is preferred to protect the group with a protective group in order to inhibit an abrupt reaction with fluorine gas. As the protective group, use can be made of generally known ones for respective substituents. Examples thereof include esters for carboxyl and hydroxyl groups, imides for amino and amide groups, and indole for guanidyl group.

E¹ is -COX, -SO₂X, or -C(R^{E11})(R^{E12})OH.

X is a hydroxyl group or a halogen atom, and is preferably a halogen atom from the standpoint that this eliminates the need of using a condensing agent in the reaction with compound (4). The halogen atom is preferably a chlorine atom or a fluorine atom, more preferably a fluorine atom.

R^{E11} and R^{E12} are each independently a hydrogen atom or a monovalent organic group having 1-10 carbon atoms. The monovalent organic group is preferably a monovalent hydrocarbon group or a monovalent hydrocarbon group containing etheric oxygen atom between carbon atoms, and is more preferably an alkyl group or an alkyl group containing an etheric oxygen atom between carbon atoms.

The monovalent organic group having 1-10 carbon atoms is preferably a monovalent hydrocarbon group having 1-10 carbon atoms or a monovalent hydrocarbon group having 2-10 carbon atoms which contains an etheric oxygen atom between carbon atoms, and is more preferably an alkyl group having 1-10 carbon atoms, from the standpoint that compound (1) can be produced at low cost.

Compound (1) is preferably the following compound (1a) or compound (1b).

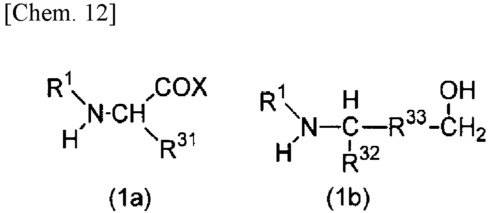

R¹ is the same as defined above.

R³¹ and R³² are each independently a hydrogen atom or a monovalent organic group having 1-19 carbon atoms, preferably a monovalent organic group having 1-19 carbon atoms.

The monovalent organic group is preferably a monovalent hydrocarbon group. The number of carbon atoms of the monovalent organic group is preferably 1-10. The monovalent organic group may have a substituent.

The monovalent organic group having 1-19 carbon atoms is preferably a monovalent hydrocarbon group which has 1-10 carbon atoms and may have a substituent. Examples of the substituent include a carboxyl group, an amino group, a benzene ring, a heterocycle, a hydroxyl group, a thiol group, a thioether group, an amide group, an imino group, and a guanidyl group. Preferred as the heterocycle are nitrogen-containing heterocycles, and especially preferred are a pyrrole ring, an imidazole ring, and an indole ring. From the standpoints of inhibiting an abrupt reaction with fluorine gas and improving the solubility in a solvent in the fluorination reaction, some or all of the hydrogen atoms of R³¹ and R³² may have been replaced by fluorine atoms. In the case where R³¹ and R³² have substituents, the number of carbon atoms of the substituents is included in the number of carbon atoms of R³¹ and R³².

When R¹ is not a hydrogen atom, the R¹ may be bonded to R³¹ to form a divalent organic group. In this case, the divalent organic group formed by the bonding of the R¹ to R³¹ is a divalent hydrocarbon group having 1-20 carbon atoms or a divalent hydrocarbon group having 2-20 carbon atoms which contains an etheric oxygen atom between carbon atoms.

When R¹ is not a hydrogen atom, the R¹ may be bonded to R³² to form a divalent organic group. In this case, the divalent organic group formed by the bonding of the R¹ to R³² is a divalent hydrocarbon group having 1-20 carbon atoms or a divalent hydrocarbon group having 2-20 carbon atoms which contains an etheric oxygen atom between carbon atoms.

R³³ is a single bond or a divalent organic group having 1-10 carbon atoms.

The divalent organic group is preferably a divalent hydrocarbon group or a divalent hydrocarbon group containing an etheric oxygen atom between carbon atoms, and is more preferably an alkylene group or an alkylene group containing an etheric oxygen atom between carbon atoms. From the standpoints of inhibiting an abrupt reaction with fluorine gas and improving the solubility in a solvent in the fluorination reaction, some or all of the hydrogen atoms of R³³ may have been replaced by fluorine atoms.

The divalent organic group having 1-10 carbon atoms is preferably a divalent hydrocarbon group having 1-10 carbon atoms or a divalent hydrocarbon group having 2-10 carbon atoms which contains an etheric oxygen atom between carbon atoms, and is more preferably an alkylene group having 1-10 carbon atoms, a (per)fluoroalkylene group having 1-10 carbon atoms, -CH₂(OCH₂CH₂)ₜOCH₂- (t = 1-3), or -CH₂CH₂(OCH₂CH₂)ₜOCH₂CH₂-(t = 1-3).

The total number of carbon atoms of R³² and R³³ is 0-19.

More preferred as compound (1a) are the following compounds (amino acids).

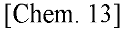

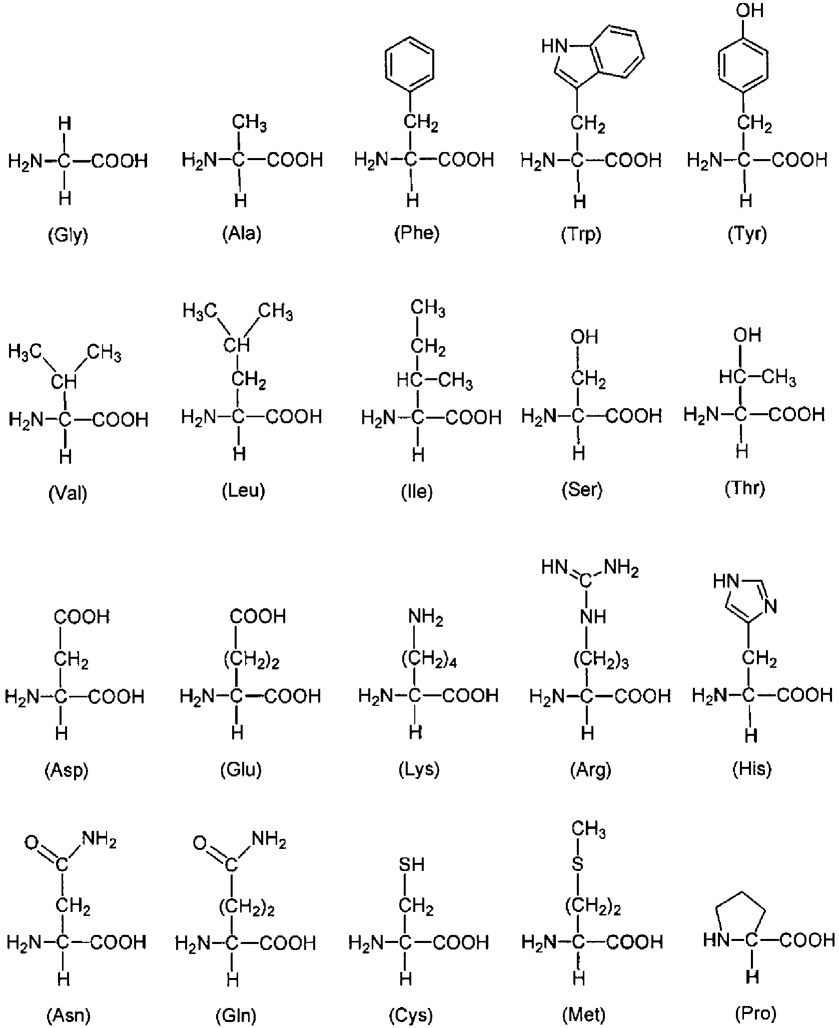

More preferred as compound (1b) are the following compounds (amino alcohols).

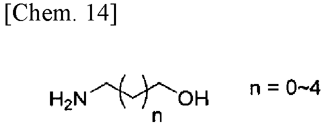

### <Compound (2)>

Compound (2) is not particularly limited so long as it is a compound having an amino-protecting group. Compound (2) is preferably a compound having an electron-withdrawing group. It is preferable that compound (2) contains a group including an electron-withdrawing group. The group including an electron-withdrawing group is preferably Z¹-CO-, Z²-O-CO-, or Z¹-SO₂-, especially preferably Z¹-CO- or Z¹-SO₂-. The electron-withdrawing group is preferably -CO-, -O-CO-, or -SO₂-. Z¹ is a halogen atom, a monovalent hydrocarbon group which has 1-10 carbon atoms and in which some or all of the hydrogen atoms may have been replaced by halogen atoms, or a monovalent hydrocarbon group which has 2-10 carbon atoms and contains an etheric oxygen atom between carbon atoms and in which some or all of the hydrogen atoms may have been replaced by halogen atoms. The halogen atom is preferably a fluorine atom. Z² is a monovalent hydrocarbon group which has 1-10 carbon atoms and in which some or all of the hydrogen atoms may have been replaced by halogen atoms or a monovalent hydrocarbon group which has 2-10 carbon atoms and contains an etheric oxygen atom between carbon atoms and in which some or all of the hydrogen atoms may have been replaced by halogen atoms. In the case where the protective group has high electron-withdrawing properties, the linkage between the nitrogen atom bonded to the protective group and the carbon atom adjoining the nitrogen atom is rendered strong and, hence, the substrate is inhibited from decomposing during fluorination reaction, resulting in an improvement in yield. From this standpoint, it is preferable that Z¹ and Z² are each a monovalent perfluorohydrocarbon group having 1-10 carbon atoms or a monovalent perfluorohydrocarbon group having 2-10 carbon atoms which contains an etheric oxygen atom between carbon atoms.

In the case where compound (2) is one which has a plurality of groups each including an electron-withdrawing group and in which Z¹ is not a halogen atom, then the Z¹ moieties or the Z² moieties may be bonded to each other to form a divalent organic group. In this case, the divalent organic group formed by the bonding between the Z¹ moieties or between the Z² moieties is a divalent hydrocarbon group which has 1-10 carbon atoms and in which some or all of the hydrogen atoms may have been replaced by halogen atoms or a divalent hydrocarbon group which has 2-10 carbon atoms and contains an etheric oxygen atom between carbon atoms and in which some or all of the hydrogen atoms may have been replaced by halogen atoms.

A compound obtained by reacting compound (1) with compound (2) in which Z¹ moieties or Z² moieties have been bonded to each other to form a divalent organic group corresponds to compound (3)-1, which is described later, in which R⁴ and R⁵ are each a monovalent organic group including an electron-withdrawing group and have been bonded to each other to form a divalent organic group.

Especially preferred as compound (2) are the following compounds.

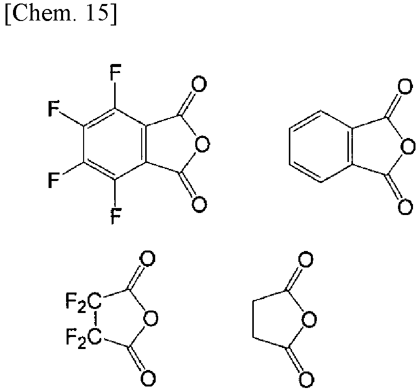

CF₃CF₂[CF₂OCF(CF₃)]ₛCOF (s = 1-3)

The reaction between compound (1) and compound (2) can be conducted using a known reaction method and known conditions in accordance with the structure of the protective group of compound (2). In the case of using a solvent, examples thereof include toluene, dichloromethane, chloroform, triethylamine, dichloropentafluoropropane (trade name AK-225, manufactured by AGC), and tetrahydrofuran. Two or more thereof may be used. Preferred reaction temperatures are from -10°C to the boiling temperature of the solvent.

### <Compound (3)-1>

The reaction between compound (1) and compound (2) yields compound (3)-1, which contains a protected amino group. In the case where R¹ and/or R² is a hydrogen atom, the hydrogen atom is converted to a group including an electron-withdrawing group. In the case where R¹ or R² is not a hydrogen atom, this group remains as such. That is, compound (3)-1 has at least one group including an electron-withdrawing group.

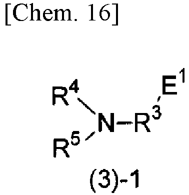

R³ and E¹ are each the same as defined above.

R⁴ and R⁵ are each independently a monovalent organic group including an electron-withdrawing group, a monovalent hydrocarbon group having 1-20 carbon atoms, or a monovalent hydrocarbon group having 2-20 carbon atoms which contains an etheric oxygen atom between carbon atoms.

At least one of R⁴ and R⁵ is a monovalent organic group including an electron-withdrawing group.

R³, R⁴, and R⁵ may be bonded to each other to form a divalent organic group or a trivalent organic group. Specifically, R³ and R⁴ may be bonded to each other, R⁴ and R⁵ may be bonded to each other, R³ and R⁵ may be bonded to each other, or R³, R⁴, and R⁵ may be bonded to each other, each thereby forming a ring.

In this case, the group formed by the bonding between R³ and R⁴ is a divalent organic group having 1-20 carbon atoms. The group formed by the bonding between R⁴ and R⁵ is a divalent organic group including an electron-withdrawing group. The group formed by the bonding between R³ and R⁵ is a divalent organic group having 1-20 carbon atoms. The group formed by the bonding between R³, R⁴, and R⁵ is a trivalent organic group having 1-20 carbon atoms.

At least one of R⁴ and R⁵ is a monovalent organic group including an electron-withdrawing group. In the case where the substituents on the nitrogen atom are deficient in electron, the substrate is more inhibited from decomposing during fluorination reaction and an improvement in yield can be attained. From this standpoint, it is preferable that R⁴ and R⁵ are each a monovalent organic group including an electron-withdrawing group.

From the same standpoint, it is preferable that R⁴ and R⁵ are bonded to each other to form a divalent organic group including an electron-withdrawing group.

That is, it is especially preferable that R⁴ and R⁵ are each a monovalent organic group including an electron-withdrawing group and are bonded to each other to form a ring.

In the case where R¹ or R² is not a hydrogen atom, this group remains as such.
This R⁴ or R⁵ is hence the same group as R¹ or R². Consequently, in the case where R⁴ and R⁵ are each a monovalent hydrocarbon group having 1-20 carbon atoms or a monovalent hydrocarbon group having 2-20 carbon atoms which contains an etheric oxygen atom between carbon atoms, then preferred examples thereof are the same as the preferred examples of R¹ or R².

The monovalent organic group including an electron-withdrawing group is not particularly limited so long as it is a group functioning as an amino-protecting group. This monovalent organic group is more preferably Z¹-CO-, Z²-O-CO-, or Z¹-SO₂-, especially preferably Z¹-CO- or Z¹-SO₂-. The electron-withdrawing group is preferably -CO-, -O-CO-, or -SO₂-.

Z¹ is a halogen atom, a monovalent hydrocarbon group which has 1-10 carbon atoms and in which some or all of the hydrogen atoms may have been replaced by halogen atoms, or a monovalent hydrocarbon group which has 2-10 carbon atoms and contains an etheric oxygen atom between carbon atoms and in which some or all of the hydrogen atoms may have been replaced by halogen atoms. The halogen atom is preferably a fluorine atom. Z² is a monovalent hydrocarbon group which has 1-10 carbon atoms and in which some or all of the hydrogen atoms may have been replaced by halogen atoms or a monovalent hydrocarbon group which has 2-10 carbon atoms and contains an etheric oxygen atom between carbon atoms and in which some or all of the hydrogen atoms may have been replaced by halogen atoms. In the case where the substituents on the nitrogen atom are deficient in electron, the linkage between the nitrogen atom, which has, bonded thereto, a group including an electron-withdrawing group, and the carbon atom adjoining the nitrogen atom is stronger (it is preferable that the electron-withdrawing group has been directly bonded to the nitrogen atom). This compound (3)-1 is hence inhibited from abruptly reacting with fluorine gas and has improved solubility in a solvent in fluorination reaction. As a result, the substrate is inhibited from decomposing during the fluorination reaction and an improvement in yield can be attained. From this standpoint, it is preferable that Z¹ and Z² are each a monovalent perfluorohydrocarbon group having 1-10 carbon atoms or a monovalent perfluorohydrocarbon group having 2-10 carbon atoms which contains an etheric oxygen atom between carbon atoms.

In the case where compound (3)-1 is one which has a plurality of groups each including an electron-withdrawing group and in which Z¹ is not a halogen atom, then the Z¹ moieties or the Z² moieties may be bonded to each other to form a divalent organic group. In this case, the group formed by the bonding between the Z¹ moieties or between the Z² moieties is a divalent hydrocarbon group which has 1-10 carbon atoms and in which some or all of the hydrogen atoms may have been replaced by halogen atoms or a divalent hydrocarbon group which has 2-10 carbon atoms and contains an etheric oxygen atom between carbon atoms and in which some or all of the hydrogen atoms may have been replaced by halogen atoms. This compound (3)-1 is a compound in which R⁴ and R⁵, which are groups each including an electron-withdrawing group, have been bonded to each other to form such divalent organic group.

Shown below are especially preferred structures of (R⁴)(R⁵)N- in which R⁴ and R⁵ have been bonded to each other to form a divalent organic group. In the following, Z³ is a divalent hydrocarbon group which has 1-10 carbon atoms and in which some or all of the hydrogen atoms may have been replaced by halogen atoms or a divalent hydrocarbon group which has 2-10 carbon atoms and contains an etheric oxygen atom between carbon atoms and in which some or all of the hydrogen atoms may have been replaced by halogen atoms. Examples of the halogen atoms include fluorine atoms. Z³ may include one or two fused rings, and examples of the fused rings include aromatic rings, 4- to 6-membered saturated carbon rings, and 4- to 6-membered unsaturated carbon rings. Some or all of the hydrogen atoms of these fused rings may be replaced by halogen atoms, and the fused rings may have been substituted with an alkyl group having 1-5 carbon atoms or with a halogenoalkyl group having 1-5 carbon atoms. Examples of the halogen atoms include fluorine atoms.

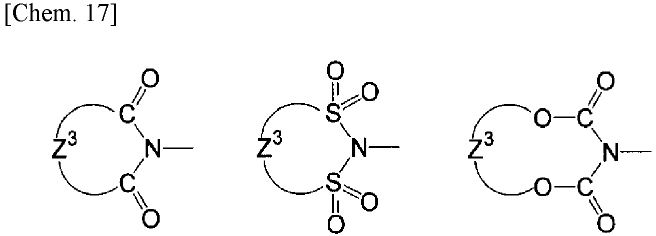

Especially preferred structures of (R⁴)(R⁵)N- are shown below. Z¹ is the same as defined above.

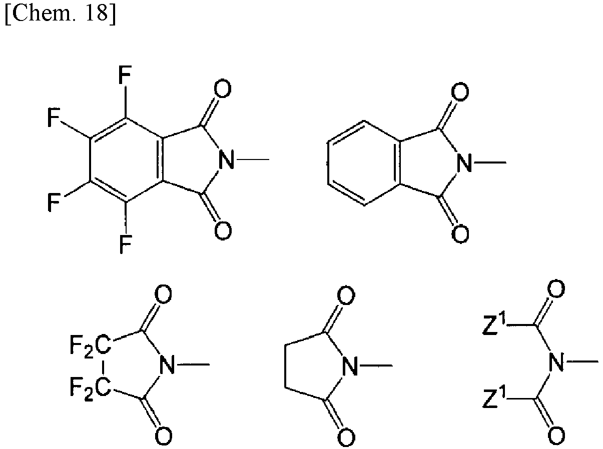

Compound (3)-1 is preferably the following compound (3a)-1 or compound (3b)-1.

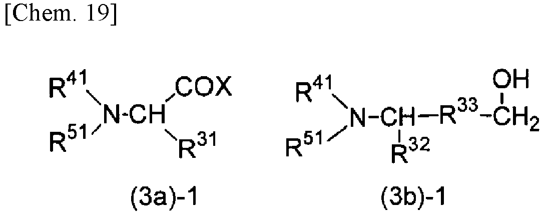

R³¹, R³², R³³, and X are the same as defined above.

R⁴¹ is a monovalent organic group including an electron-withdrawing group, a monovalent hydrocarbon group having 1-20 carbon atoms, or a monovalent hydrocarbon group having 2-20 carbon atoms which contains an etheric oxygen atom between carbon atoms.

R⁵¹ is a monovalent organic group including an electron-withdrawing group.

The monovalent organic group including an electron-withdrawing group, the monovalent hydrocarbon group having 1-20 carbon atoms, or the monovalent hydrocarbon group having 2-20 carbon atoms which contains an etheric oxygen atom between carbon atoms is the same as in R⁴ and R⁵, and preferred examples thereof are also the same.

R³¹, R⁴¹, and R⁵¹ may be bonded to each other to form a divalent organic group or a trivalent organic group. Specifically, R³¹ and R⁴¹ may be bonded to each other, R⁴¹ and R⁵¹ may be bonded to each other, R³¹ and R⁵¹ may be bonded to each other, or R³¹, R⁴¹, and R⁵¹ may be bonded to each other, each thereby forming a ring.

In this case, the group formed by the bonding between R³¹ and R⁴¹ is a divalent organic group having 1-20 carbon atoms. The group formed by the bonding between R⁴¹ and R⁵¹ is a divalent organic group including an electron-withdrawing group. The group formed by the bonding between R³¹ and R⁵¹ is a divalent organic group having 1-20 carbon atoms. The group formed by the bonding between R³¹, R⁴¹, and R⁵¹ is a trivalent organic group having 1-20 carbon atoms.

R³², R⁴¹, and R⁵¹ may be bonded to each other to form a divalent organic group or a trivalent organic group. Specifically, R³² and R⁴¹ may be bonded to each other, R⁴¹ and R⁵¹ may be bonded to each other, R³² and R⁵¹ may be bonded to each other, or R³², R⁴¹, and R⁵¹ may be bonded to each other, each thereby forming a ring.

In this case, the group formed by the bonding between R³² and R⁴¹ is a divalent organic group having 1-20 carbon atoms. The group formed by the bonding between R⁴¹ and R⁵¹ is a divalent organic group including an electron-withdrawing group. The group formed by the bonding between R³² and R⁵¹ is a divalent organic group having 1-20 carbon atoms. The group formed by the bonding between R³², R⁴¹, and R⁵¹ is a trivalent organic group having 1-20 carbon atoms.

### <Compound (4)>

E² of compound (4) reacts with E¹ of compound (1) to form linking group E³, thereby linking R⁶ to compound (1).

[Chem. 20] E²-R⁶ (4)

E² is -C(R^{E21})(R^{E22})OH, -COX, or -SO₂X.

R^{E21} and R^{E22} are each a hydrogen atom or a monovalent organic group having 1-20 carbon atoms. Preferred examples of the monovalent organic group having 1-20 carbon atoms are the same as those of R⁶, which is described later.

X is a hydroxyl group or a halogen atom, and is preferably a halogen atom from the standpoint that this eliminates the need of using a condensing agent in the reaction with compound (1). The halogen atom is preferably a chlorine atom or a fluorine atom, more preferably a fluorine atom.

E² reacts with E¹ to form linking group E³. It is hence preferable that when E¹ is -COX or -SO₂X, then E² is -C(R^{E21})(R^{E22})OH and that when E¹ is -C(R^{E11})(R^{E12})OH, then E² is -COX or -SO₂X.

R⁶ is a monovalent organic group having 1-20 carbon atoms. R⁶ is a substituent to be introduced into compound (5), which is a starting material to be subjected to fluorination reaction. The larger the proportion of fluorine atoms contained in R⁶, the higher the solubility of compound (5) in a fluorination solvent to be used in the fluorination reaction and the more the fluorination yield can be improved. It is hence preferable that some of the hydrogen atoms of R⁶ have been replaced by fluorine atoms, and it is preferable that all of the hydrogen atoms have been replaced by fluorine atoms. Acceptable heteroatoms other than fluorine atoms are chlorine atoms and etheric oxygen atoms. The number of heteroatoms in R⁶ is preferably 3 or less.

The monovalent organic group is preferably a monovalent saturated hydrocarbon group. The number of carbon atoms of R⁶ is preferably 1-12.

Consequently, R⁶ is preferably a group which is selected from a monovalent saturated hydrocarbon group having 1-20 carbon atoms, a partially halogenated, monovalent saturated hydrocarbon group having 1-20 carbon atoms, a monovalent saturated hydrocarbon group having 2-20 carbon atoms that contains an etheric oxygen atom between carbon atoms, and a partially halogenated, monovalent saturated hydrocarbon group having 2-20 carbon atoms that contains an etheric oxygen atom between carbon atoms, and in which at least some, preferably all, of the hydrogen atoms have been replaced by fluorine atoms. R⁶ is especially preferably a perfluoroalkyl group having 1-20 carbon atoms or a perfluoro(alkoxyalkyl) group having 1-20 carbon atoms, and is most preferably a perfluoroalkyl group having 1-12 carbon atoms or a perfluoro(alkoxyalkyl) group having 3-12 carbon atoms.

R⁶ is more preferably R⁶¹ or R⁶², which is described later.

Compound (4) is preferably the following compound (4a) when the starting material is compound (1a), and is preferably the following compound (4b) when the starting material is compound (1b).

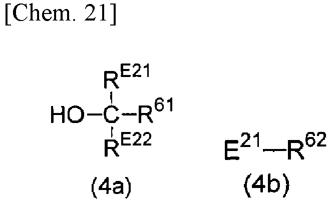

R^{E21} and R^{E22} are the same as defined above.

R⁶¹ is a monovalent organic group having 1-20 carbon atoms.

E²¹ is -COX or -SO₂X. X is a hydroxyl group or a halogen atom. The halogen atom can be a chlorine atom or a fluorine atom, and is preferably a fluorine atom.

R⁶² is a monovalent organic group having 1-20 carbon atoms.

Preferred examples of R⁶¹ are the same as the preferred examples of R⁶ shown above. More preferred are the following structures.

-(CF₂)ᵤCF₃ (u = 0-7)

-[CF(CF₃)OCF₂]ᵥCF₂CF₃ (v=1-10)

Especially preferred structures of R⁶¹ are as follows.

-CF₃

-CF₂CF₃

-(CF₂)₆CF₃

-(CF₂)₇CF₃

-CF(CF₃)OCF₂CF₂CF₃

-CF(CF₃)OCF₂CF(CF₃)OCF₂CF₂CF₃

-CF(CF₃)OCF₂CF(CF₃)OCF₂CF(CF₃)OCF₂CF₂CF₃

Preferred examples of compound (4a) are the following compounds.

HOCH₂(CF₂)ᵤCF₃ (u=0-7)

HOCH₂[CF(CF₃)OCF₂]ᵥCF₂CF₃ (v = 1-10)

HOCH(CF₃)₂

HOCH(CF(CF₃)₂)CF₂CF₃

HOCH(CF(CF₃)₂)₂

More preferred examples of compound (4a) are the following compounds.

HOCH₂CF₂CF₃

HOCH₂(CF₂)₆CF₃

HOCH₂(CF₂)₇CF₃

HOCH₂CF(CF₃)OCF₂CF₂CF₃

HOCH₂CF(CF₃)OCF₂CF(CF₃)OCF₂CF₂CF₃

HOCH₂CF(CF₃)OCF₂CF(CF₃)OCF₂CF(CF₃)OCF₂CF₂CF₃

Preferred examples of R⁶² are the same as the preferred examples of R⁶ shown above. More preferred are the following structures.

-(CF₂)ᵤCF₃ (u = 0-7)

-[CF(CF₃)OCF₂]ᵥCF₂CF₃ (v=1-10)

Especially preferred structures of R⁶² are as follows.

-CF₂CF₃

-(CF₂)₆CF₃

-CF(CF₃)OCF₂CF₂CF₃

-CF(CF₃)OCF₂CF(CF₃)OCF₂CF₂CF₃

-CF(CF₃)OCF₂CF(CF₃)OCF₂CF(CF₃)OCF₂CF₂CF₃

The reaction between compound (3)-1 and compound (4) can be conducted using a known reaction method and known conditions in accordance with the structures of E¹ and E² and a combination thereof.

For example, in the case where E¹ is -COX and E² is -C(R^{E21})(R^{E22})OH or when E¹ is -C(R^{E11})(R^{E12})OH and E² is -COX or -SO₂X, then the reaction can be conducted under known reaction conditions. In the case of using a solvent, examples thereof include methylene chloride, dichloromethane, chloroform, triethylamine, tetrahydrofuran, AK-225, fluorinated alkanes (e.g., C₆F₁₃H (ASAHI KLIN (registered trademark) AC-2000, manufactured by AGC Inc.), C₆F₁₃C₂H₅ (ASAHI KLIN (registered trademark) AC-6000, manufactured by AGC Inc.), and C₂F₅CHFCHFCF₃ (Vertrel (registered trademark) XF, manufactured by The Chemours Co.)), and fluoroalkyl ethers (e.g., CH₂OCF₂CF₂H (ASAHI KLIN (registered trademark) AE-3000, manufactured by AGC Inc.), C₄F₉OCH₃ (Novec (registered trademark) 7100, manufactured by 3M Ltd.), C₄F₉OC₂H₅ (Novec (registered trademark) 7200, manufactured by 3M Ltd.), and C₂F₅CF(OCH₃)C₃F₇ (Novec (registered trademark) 7300, manufactured by 3M Ltd.). Two or more thereof may be used. The reaction temperature is usually preferably -50°C or higher, and is preferably +100°C or lower or not higher than the boiling temperature of the solvent. The reaction time for this reaction can be appropriately changed in accordance with the rates of feeding the starting materials and with the amounts of the compounds to be used in the reaction. The reaction pressure (gauge pressure; the same applies hereinafter) is preferably 0-2 MPa.

Even in the case where an acid (HX) generates as a by-product of the reaction, the reaction product does not form a salt with the acid because the amino group has been protected with a group including an electron-withdrawing group (it is preferable that the electron-withdrawing group has been directly bonded to the nitrogen atom). It is hence unnecessary to use a base for catching the acid. A base can be used in order to protect the reaction vessel or for other purposes. In this case, the base may be either an organic base or an inorganic base. Examples of the organic base include diethylamine, triethylamine, and pyridine. Preferred are amines having no active hydrogen, and triethylamine and pyridine are preferred. Examples of the inorganic base include sodium hydroxide, potassium hydroxide, sodium hydrogen carbonate, potassium carbonate, and sodium fluoride. Sodium fluoride is preferred.

A crude reaction product including compound (5) yielded by the reaction between compound (3)-1 and compound (4) may be purified in accordance with an intended use or may be subjected as such to the next reaction, etc. From the standpoint of safely performing fluorination reaction in the next step, it is usually desirable to purify the crude reaction product.

In the case of purifying the crude reaction product, examples include a method in which the crude reaction product is distilled as such, a method in which the crude reaction product is treated with a dilute aqueous alkali solution or the like and the mixture is subjected to liquid separation, a method in which the crude reaction product is extracted with an appropriate organic solvent and the extract is distilled, and silica gel column chromatography.

### <Compound (3)-2>

By reacting compound (1) first with compound (4), compound (3)-2 is obtained.

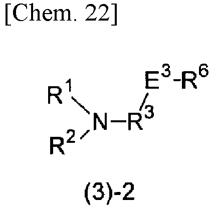

R¹, R², R³, and R⁶ are each the same as defined above.

E³ is a divalent organic group formed by a reaction between E¹ and E², and is preferably -COO-C(R^{E21})(R^{E22})-, -SO₂-C(R^{E21})(R^{E22})-, -C(R^{E11})(R^{E12})-O-C(=O)-, or -C(R^{E11})(R^{E12})-SO₂-.

Compound (3)-2 is preferably the following compound (3a)-2 or compound (3b)-2.

Compound (3a)-2 is obtained by a reaction between compound (1a) and compound (4a).

Compound (3b)-2 is obtained by a reaction between compound (1b) and compound (4b).

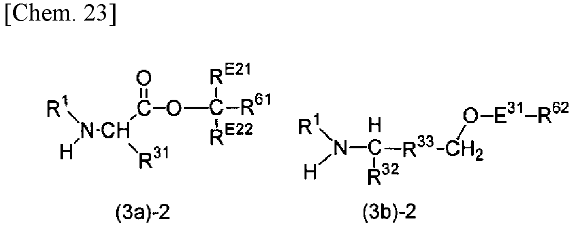

R¹, R³, R³², R³³, R^{E21}, R^{E22}, R⁶¹, and R⁶² are the same as defined above.

E³¹ is -CO- or -SO₂-.

Conditions for the reaction between compound (1) and compound (4) are the same as the conditions for the reaction between compound (3)-1 and compound (4).

By reacting compound (3)-2 with compound (2) to protect the amino group, compound (5) is obtained.

Conditions for the reaction between compound (3)-2 and compound (2) are the same as the conditions for the reaction between compound (1) and compound (2).

### <Compound (5)>

Compound (5) is a starting material to be subjected to fluorination reaction in the production method of the present invention. HF, which generates as a by-product of the fluorination reaction, is prone to form hydrogenfluorides with amine compounds, and the hydrogenfluorides reduce the solubility of the amine compounds in a solvent for the fluorination reaction. Since the amino group has been protected by a group including an electron-withdrawing group, the nitrogen atom is in an electron-deficient state and compound (5) has reduced reactivity with the HF (it is preferable that the electron-withdrawing group has been directly bonded to the nitrogen atom). It is thought that the amine compound is thereby inhibited from decreasing in solubility and, as a result, the fluorination reaction proceeds with high yield.

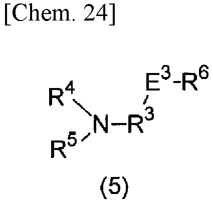

R³ to R⁶ and E³ are each the same as defined above.

From the standpoint of smoothly conducting the fluorination reaction in a liquid phase, compound (5) preferably has a molecular weight of 200-1,000. In case where the molecular weight thereof is too low, this compound (5) is prone to vaporize and it is hence likely that the fluorination reaction in the liquid phase is accompanied with a decomposition reaction in the gas phase. Meanwhile, in case where the molecular weight thereof is too high, this compound (5) is likely to be difficult to handle.

It is preferable that compound (5) is a compound containing fluorine atoms, from the standpoint of rendering compound (5) easy to dissolve in a liquid phase to be used for the fluorination reaction. The fluorine content of compound (5) (proportion of fluorine atoms in the molecule) is preferably 10 mass% or higher, more preferably 10-86 mass%, still more preferably 10-76 mass%, especially preferably 30-76 mass%.

Compound (5) is preferably the following compound (5a) or compound (5b).

Compound (5a) is obtained, for example, from the compound (1a) as a starting material by the method described above.

Compound (5b) is obtained, for example, from the compound (1b) as a starting material by the method described above.

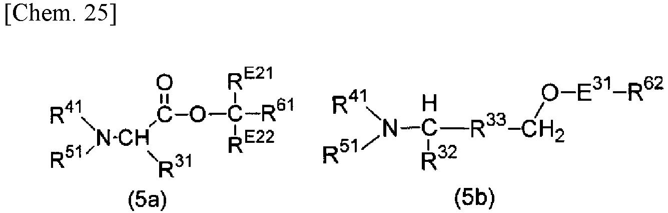

R³¹, R³², R³³, R^{E21}, R^{E22}, R⁴¹, R⁵¹, R⁶¹, R⁶², and E³¹ are each the same as defined above.

More preferred examples of compound (5a) are the following compounds.

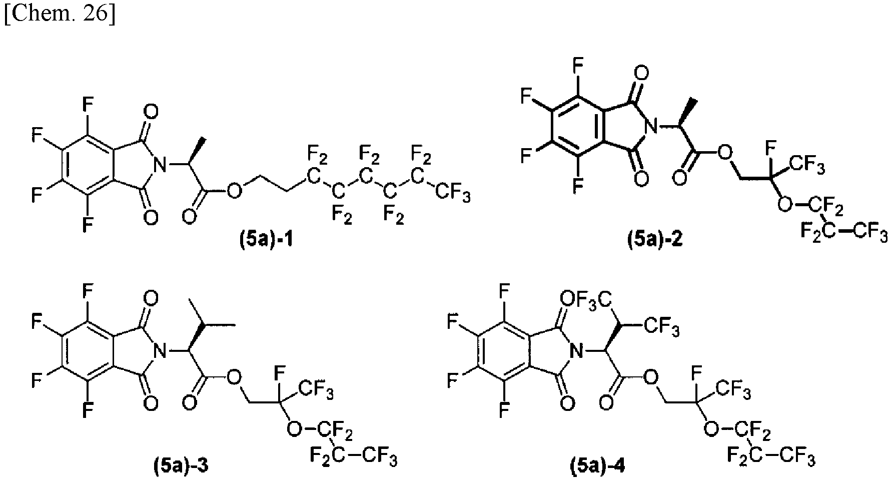

A more preferred example of compound (5b) is the following compound.

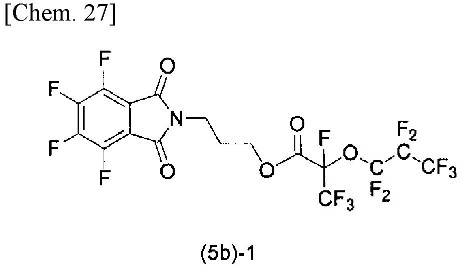

### «Fluorination Reaction»

Compound (5) is fluorinated to thereby obtain compound (6).

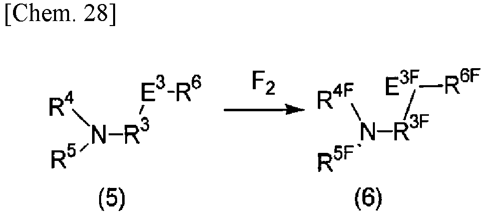

R^{3F} is a group formed by fluorinating some or all of the hydrogen atoms of R³.

R^{4F} is a group formed by fluorinating some or all of the hydrogen atoms of R⁴.

R^{5F} is a group formed by fluorinating some or all of the hydrogen atoms of R⁵.

R^{6F} is a group formed by fluorinating some or all of the hydrogen atoms of R⁶.

E^{3F} is a group formed by fluorinating some or all of the hydrogen atoms of E³.

The fluorination reaction of compound (5) can be in theory conducted even by a cobalt fluorination method, an ECF method, or a gas-phase fluorination method. However, fluorination in a liquid phase is an especially advantageous method from the standpoints of reaction yield and reaction operation. The fluorination in a liquid phase is conducted by a method in which compound (5) is reacted with fluorine in a liquid phase. As the fluorine, fluorine gas may be used as such or fluorine gas diluted with an inert gas may be used. The inert gas is preferably nitrogen gas or helium gas, and is especially preferably nitrogen gas for reasons of profitability. The fluorine gas content of the nitrogen gas is not particularly limited. In general, the higher the proportion of fluorine gas, the higher the reactivity. Consequently, in the case where hydrogen atoms having low reactivity are desired to be fluorinated or when the reaction rate is desired to be heightened, then the content of fluorine gas is preferably 10 vol% or higher, especially preferably 20 vol% or higher. In the case where milder reaction conditions are employed in order to inhibit the starting material and the product from decomposing and to thereby attain an improvement in yield or when lowly reactive hydrogen atoms are left unfluorinated on purpose and the other hydrogen atoms only are desired to be fluorinated, then the content of fluorine gas is preferably 1-10 vol%, especially preferably 1-5 vol%. Hydrogen atoms bonded to tertiary carbon atoms frequently have low reactivity. Because of this, in the case of a compound including such hydrogen atom bonded to an asymmetric center, the other hydrogen atoms can all be fluorinated while leaving only the hydrogen atom on the asymmetric center unfluorinated, by employing milder reaction conditions. Thus, a fluorinated product retaining the intact asymmetry of the starting-material molecule is obtained.

The liquid phase may be one constituted of a compound itself which takes part in the reaction, but a solvent may be used as the liquid phase. Preferred fluorination solvents are: solvents each containing no C-H bond and essentially containing C-F bonds; perfluoroalkanes; and organic solvents obtained by perfluorinating known organic solvents each having in the structure one or more atoms selected from chlorine, nitrogen, and oxygen atoms. Furthermore, it is preferred to use a fluorination solvent in which compound (5) has high solubility, and it is especially preferred to use a fluorination solvent in which compound (5) can dissolve in a concentration of 1 mass% or higher, in particular, in a concentration of 5 mass% or higher.

The liquid phase in the fluorination reaction of compound (5) is preferably one including any of perfluoroalkanes (FC-72, etc.), perfluoroethers (FC-75, FC-77, etc.), perfluoropolyethers (trade names Krytox, Fomblin, Galden, Demnum, etc.), chlorofluorocarbons (R-113, trade name Fluonlube, and 1,2,3,4-tetrachloroperfluorobutane), chlorofluoroethers (CFE-419, etc.), chlorofluoropolyethers, and inert fluids (trade name Fluorinert).

The following acid fluorides also can be used as the liquid phase.

CF₃CF₂CF₂OCF(CF₃)COF

CF₃CF₂CF₂OCF(CF₃)CF₂OCF(CF₃)COF

CF₃CF₂CF₂OCF(CF₃)CF₂OCF(CF₃)CF₂OCF(CF₃)COF

The amount of the fluorination solvent is preferably at least 5 times by mass, especially preferably 10-100 times by mass, based on the amount of compound (5).

The mode of the fluorination reaction is preferably a batch process or a continuous process. From the standpoints of reaction yield and selectivity, it is especially preferred to perform fluorination method 2, which is described below, by a continuous process. Regardless of whether the reaction is conducted by a batch process or a continuous process, the fluorine gas to be used may be one diluted with an inert gas, e.g., nitrogen. [Fluorination Method 1] A method in which compound (5) and a solvent are introduced into a reaction vessel and stirring is initiated, and the compound (5) is reacted at a given reaction temperature and a given reaction pressure while continuously supplying fluorine gas. [Fluorination Method 2] A method in which a solvent is introduced into a reaction vessel and stirring is initiated, and compound (5) and fluorine gas are continuously and simultaneously supplied in a given molar ratio at a given reaction temperature and a given reaction pressure. In supplying compound (5) in fluorination method 2, the compound (5) may be supplied as such without being diluted with a solvent. In diluting compound (5) with a solvent, the amount of the solvent is preferably at least 5 times by mass, especially preferably at least 10 times by mass, the amount of the compound (5).

With respect to the amount of fluorine to be used for the fluorination reaction, it is preferred to conduct the reaction while keeping fluorine present so that the amount of the fluorine is always in excess in equivalent with respect to the hydrogen atoms contained in compound (5), regardless of whether the reaction is conducted by a batch process or a continuous process. From the standpoint of selectivity, it is especially preferred to use fluorine so that the fluorine amount is 1.5 times by equivalent (i.e., 1.5 times by mole) or larger. It is preferable that the fluorine amount is kept in excess in equivalent from the beginning to the end of the reaction.

The reaction temperature for the fluorination reaction can be varied in accordance with the structure of the divalent linking group (E³), but is preferably -60°C or higher but not higher than the boiling point of compound (5). From the standpoints of reaction yield, selectivity, and industrial practicability, the reaction temperature is more preferably -50°C to +100°C, especially preferably -20°C to +50°C.

The reaction pressure for the fluorination reaction is not particularly limited, and is especially preferably 0-2 MPa from the standpoints of reaction yield, selectivity, and industrial practicability.

It is preferred to add a compound containing a C-H bond to the reaction system, or to add a compound containing a carbon-carbon double bond (e.g., CF₃CF=CF₂ or CF₂=CF-CF=CF₂) to the reaction system, or to irradiate the reaction system with ultraviolet light, in order to cause the fluorination reaction to proceed efficiently. For example, in a batch process, it is preferable that the addition of a compound containing a C-H bond to the reaction system or irradiation with ultraviolet light is conducted in a late stage of the fluorination reaction. Thus, the compound (5) present in the reaction system can be efficiently fluorinated and the reaction yield can be remarkably improved. The period of ultraviolet irradiation is preferably 0.1-3 hours.

The compound containing a C-H bond is an organic compound other than compound (5), and is especially preferably an aromatic hydrocarbon. In particular, benzene, toluene, or the like is preferred. The amount of the compound containing a C-H bond to be added is preferably 0.1-10 mol%, especially preferably 0.1-5 mol%, with respect to the hydrogen atoms contained in compound (5).

It is preferable that the compound containing a C-H bond is added when the reaction system is in the state of containing fluorine. It is preferred to pressurize the reaction system in the case where the compound containing a C-H bond has been added thereto. The pressure for the pressurization is preferably 0.01-5 MPa.

The amount of the compound containing a carbon-carbon double bond to be added is preferably 0.1-100 mol%, especially preferably 0.1-50 mol%, with respect to the hydrogen atoms contained in compound (5).

It is preferable that the compound containing a carbon-carbon double bond is added when the reaction system is in the state of containing fluorine. It is preferred to pressurize the reaction system in the case where the compound containing a carbon-carbon double bond has been added thereto. The pressure for the pressurization is preferably 0.01-5 MPa.

In the case where a reaction for replacing hydrogen atoms by fluorine atoms has occurred in the reaction for fluorinating compound (5) in a liquid phase, then HF generates as a by-product. Since this HF is usually not caught by the compound (5), it is preferable that an HF scavenger is caused to coexist in the reaction system or an HF scavenger is brought into contact with an outlet gas at the gas outlet of the reaction vessel. The HF scavenger is, for example, preferably NaF.

A crude reaction product obtained through the fluorination reaction, which includes compound (6), may be subjected as such to the next step or may be purified to a higher purity. Examples of purification methods include a method in which the crude reaction product is distilled as such at ordinary pressure or a reduced pressure.

Thus, compound (5) is fluorinated to obtain compound (6).

Compound (6) is preferably the following compound (6a)-1, compound (6a)-2, compound (6b)-1, or compound (6b)-2.

Compound (6a)-1 and compound (6a)-2 are each obtained by fluorinating compound (5a) described above.

Compound (6b)-1 and compound (6b)-2 are each obtained by fluorinating compound (5b) described above.

Use of stricter fluorination conditions results in an increase in the proportion of compound (6a)-1 or compound (6b)-1, in which all the hydrogen atoms have been replaced by fluorine atoms. Examples of stricter fluorination conditions include an increased fluorine concentration, an elevated temperature, a heightened pressure, the irradiation with ultraviolet light, and the addition of a C-H compound such as those described above. Meanwhile, use of milder fluorination conditions such as those described above results in an increase in the proportion of compound (6a)-2 or compound (6b)-2, in which some of the hydrogen atoms remain. In compound (6a)-2 and compound (6b)-2, the lowly reactive hydrogen atoms bonded to tertiary carbon atoms remain and the asymmetry of the molecules of the starting materials is maintained.

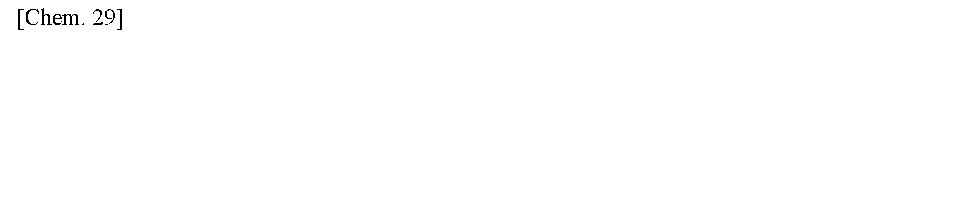

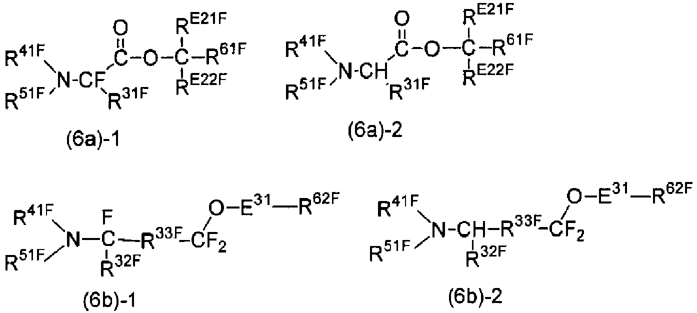

R^{31F} is a group formed by fluorinating some or all of the hydrogen atoms of R³¹.

R^{32F} is a group formed by fluorinating some or all of the hydrogen atoms of R³².

R^{33F} is a group formed by fluorinating some or all of the hydrogen atoms of R³³.

The total number of carbon atoms of R^{32F} and R^{33F} is 0-19.

R^{E21F} and R^{E22F} are each independently a fluorine atom or a group formed by fluorinating some or all of the hydrogen atoms of a monovalent organic group having 1-20 carbon atoms.

R^{41F} is a group formed by fluorinating some or all of the hydrogen atoms of R⁴¹.

R^{51F} is a group formed by fluorinating some or all of the hydrogen atoms of R⁵¹.

R^{61F} is a group formed by fluorinating some or all of the hydrogen atoms of R⁶¹.

R^{62F} is a group formed by fluorinating some or all of the hydrogen atoms of R⁶².

The following compounds are more preferred as compound (6a)-1, compound (6a)-2, compound (6b)-1, and compound (6b)-2.

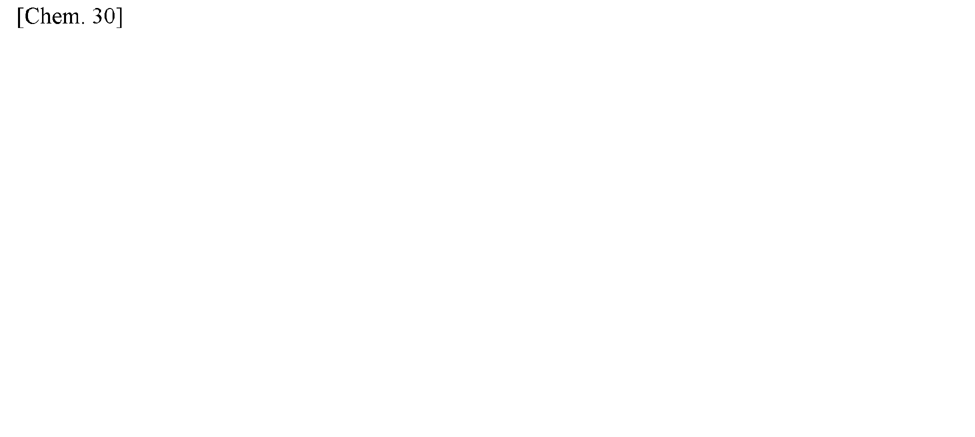

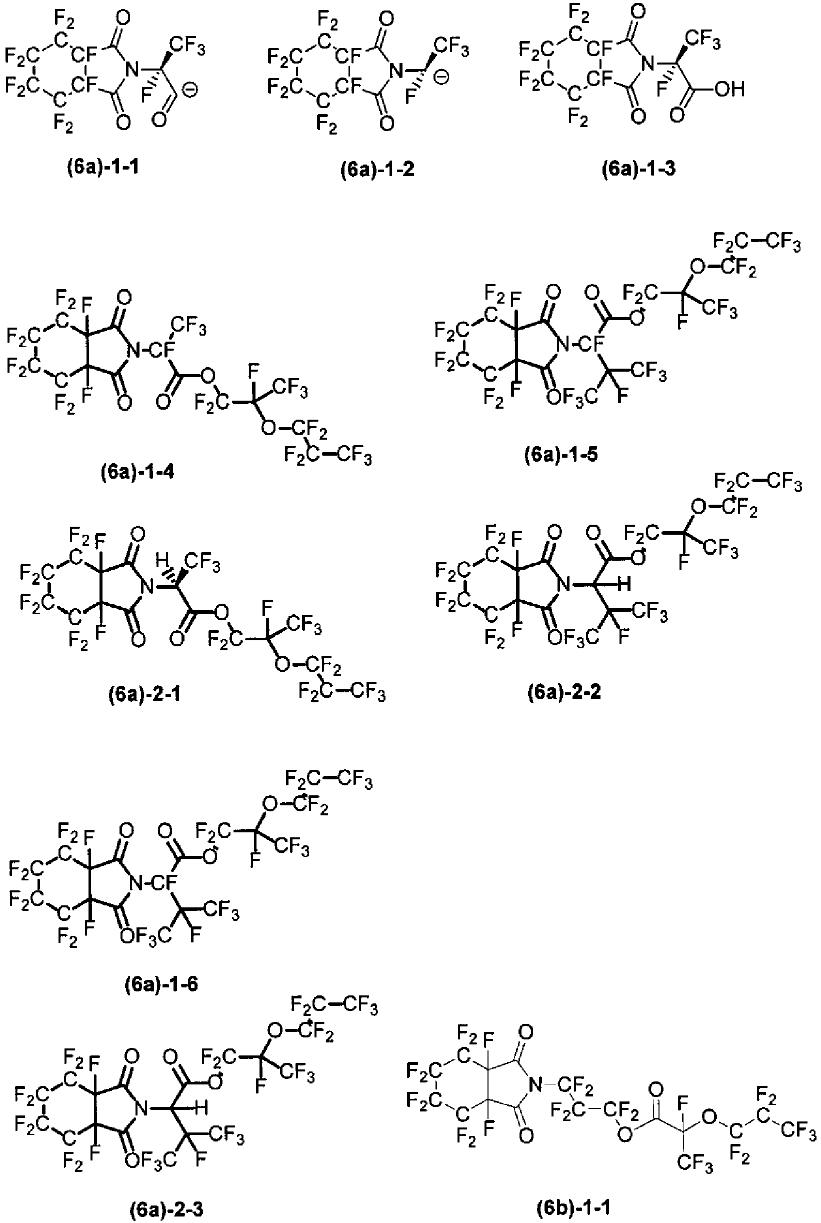

### «Deprotection and Scission»

Compound (6) is applicable for various purposes such as, for example, solvents for fluorine-containing materials, inert fluids, water-and-oil repellants, and surfactants.

Furthermore, by deprotecting the compound (6) and cutting the linking group E^{3F}, compound (7) is obtained. It is possible that compound (7) is useful as a starting material for medicines or agricultural chemicals or as a coolant for organic synthesis elements or electronic appliances.

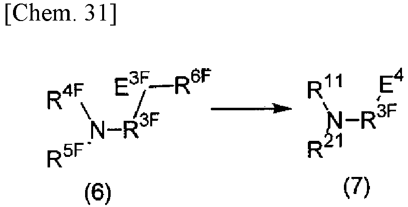

R^{3F} to R^{6F} and E^{3F} each have the same meaning as defined above.

R¹¹ and R²¹ are each independently a hydrogen atom or a monovalent organic group having 1-20 carbon atoms, at least one of R¹¹ and R²¹ being a hydrogen atom. When R¹¹ or R²¹ is not a hydrogen atom, then the R¹¹ or R²¹ may be bonded to R^{3F} to form a divalent organic group. In this case, the group formed by the bonding of the R¹¹ or R²¹ to R^{3F} is a divalent hydrocarbon group having 1-20 carbon atoms or a divalent hydrocarbon group having 2-20 carbon atoms which contains an etheric oxygen atom between carbon atoms.

E⁴ is -COX or -SO₂X. X is a hydroxyl group or a halogen atom. The halogen atom is preferably a chlorine atom or a fluorine atom, especially preferably a fluorine atom.

### Deprotection of the Amino Group:

For deprotecting the amino group, a known common technique can be employed. For example, deprotection conditions described in Protective Groups in Organic Synthesis, fifth edition (Wiley Inc.), chap. 7, Protection for the Amino Group. For example, in the case where the protective group is an amide, use can be made of a method with hydrochloric acid or hydrazine, or by hydrogenation. In the case where the protective group is an imide, in particular, a cyclic imide, the amino group can be deprotected with hydrazide, sodium boron hydride, sodium sulfide, acetic acid, hydrochloric acid, etc.

### Scission Conditions for Cutting Linking Group E^{3F}:

Examples of methods for cutting the linking group E^{3F} include a method in which the compound (6) is reacted with a nucleophilic agent in a liquid phase and decomposed thereby. The reaction may be conducted without a solvent or may be conducted in the presence of a solvent.

Examples of the solvent include inert solvents, such as perfluorotrialkylamines and perfluoronaphthalene, and a chlorotrifluoroethylene oligomer (e.g., trade name Fluonlube) which has high boiling point among chlorofluorocarbons and the like.

Examples of the nucleophilic agent include amino groups having active hydrogen (which may be ammonia, primary amines, and secondary amines), hydroxyl group, and fluorides. Preferred of these are hydroxyl group and fluorides. Examples of nucleophilic agents having a hydroxyl group include alkali hydroxides, such as sodium hydroxide, and water. Especially preferred fluorides are the fluorides of alkali metals. For example, NaF, NaHF₂, KF, and CsF are preferred. Of these, NaF is especially preferred from the standpoint of profitability. In the case where a fluoride is used as a nucleophilic agent, fluoride ions (F⁻) generate during the decomposition of the substrate and, hence, a catalytic amount suffices for the initial addition of the nucleophilic agent. The amount of the fluoride ions is preferably 1-500 mol%, more preferably 10-100 mol%, especially preferably 5-50 mol%, based on the amount of compound (6). The reaction temperature is preferably from -30°C to the boiling point of the solvent or compound (6), especially preferably -20°C to 250°C. It is preferable that this method also is carried out using a reactor equipped with a reflux column.

The sequence of performing the deprotection of the amino group and the scission of the linking group E^{3F} is not particularly limited, and either of the two may be conducted first.

Compound (7) is preferably the following compound (7a)-1, compound (7a)-2, compound (7b)-1, or compound (7b)-2. These compounds are obtained respectively from the compound (6a)-1, compound (6a)-2, compound (6b)-1, and compound (6b)-2 described above.

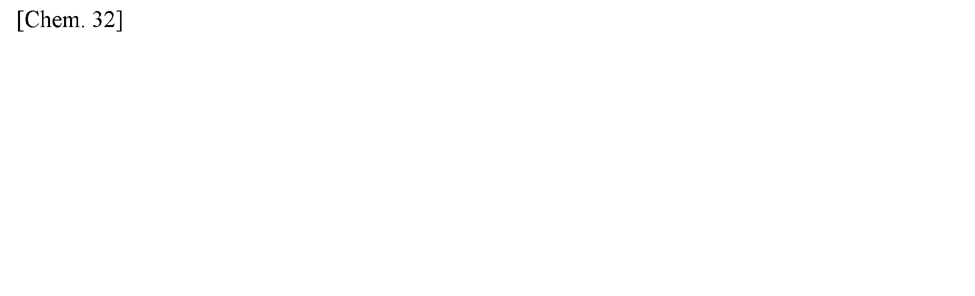

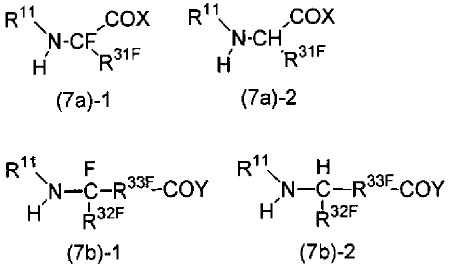

R¹¹, R^{31F}, R^{32F}, R^{33F}, and X each have the same meaning as defined above.

Y is a hydroxyl group or a halogen atom. The halogen atom is preferably a chlorine atom or a fluorine atom, more preferably a fluorine atom.

The following compounds are more preferred as compound (7a)-1, compound (7a)-2, compound (7b)-1, and compound (7b)-2.

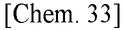

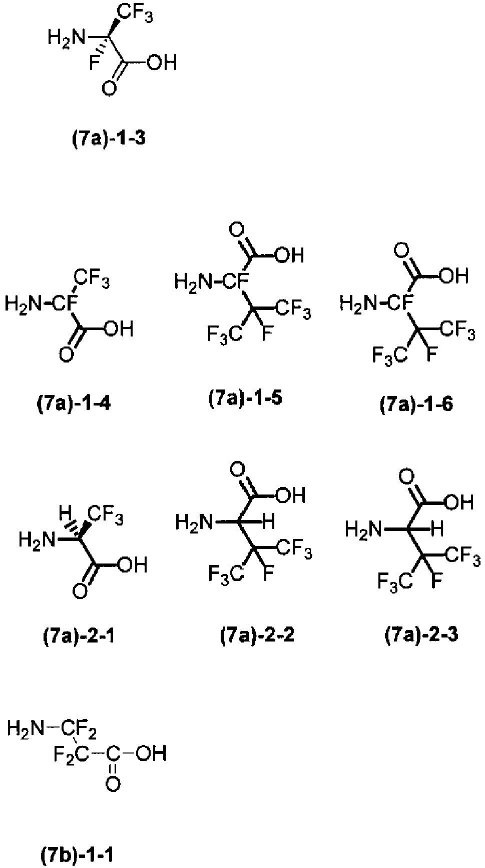

A scheme of the series of syntheses explained above is shown below, in which compound (1) is used as a starting material to obtain compound (5), which is fluorinated to obtain compound (6), which is subjected to deprotection and the scission of the linking group E^{3F} to obtain compound (7).

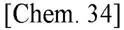

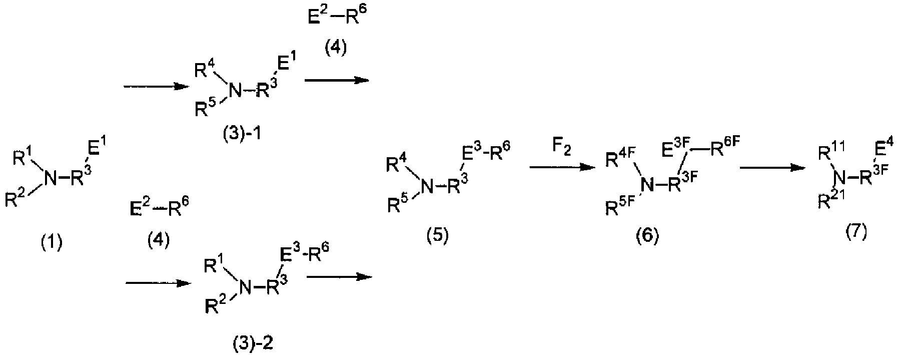

Furthermore, a scheme of a series of syntheses in which compound (1a) is used as a starting material is shown below.

A method for producing a nitrogen-containing fluorine-containing compound, the method including a step in which a compound represented by the following formula (1a) is reacted with a compound having an amino-protecting group to obtain a compound represented by the following formula (3a)-1 and
a step in which the compound represented by the following formula (3a)-1 is reacted with a compound represented by the following formula (4a) to obtain a compound represented by the following formula (5a)
or including a step in which a compound represented by the following formula (1a) is reacted with a compound represented by the following formula (4a) to obtain a compound represented by the following formula (3a)-2 and
a step in which the compound represented by the following formula (3a)-2 is reacted with a compound having an amino-protecting group to obtain a compound represented by the following formula (5a),
the method further including a step in which the compound represented by the following formula (5a) is fluorinated to obtain at least one of a compound represented by the following formula (6a)-1 and a compound represented by the following formula (6a)-2 and
a step in which at least one of the compound represented by the following formula (6a)-1 and the compound represented by the following formula (6a)-2 is subjected to deprotection and scission of the -C(=O)O- group to obtain at least one of a compound represented by the following formula (7a)-1 and a compound represented by the following formula (7a)-2.

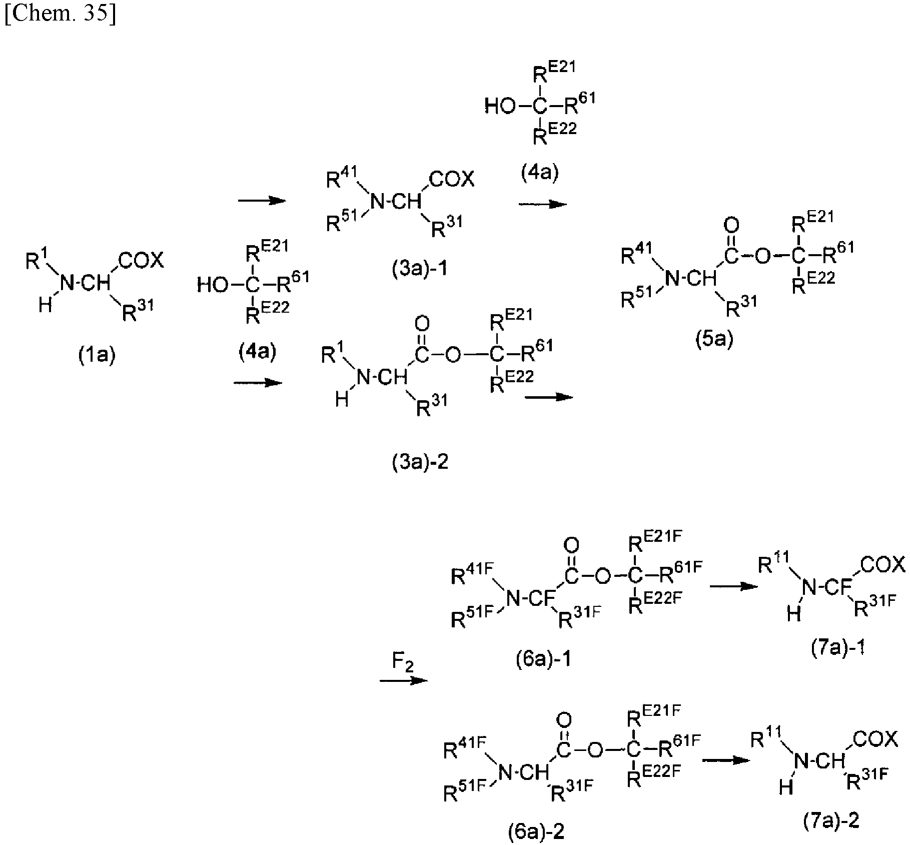

Definitions of the substituents in the formulae are as follows.

R¹ is a hydrogen atom, a monovalent hydrocarbon group having 1-20 carbon atoms, or a monovalent hydrocarbon group having 2-20 carbon atoms which contains an etheric oxygen atom between carbon atoms. When R¹ is not a hydrogen atom, the R¹ may be bonded to R³¹ to form a divalent organic group.

R³¹: a hydrogen atom or a monovalent organic group having 1-19 carbon atoms.

X: a hydroxyl group or a halogen atom.

R⁴¹: a monovalent organic group having an electron-withdrawing group, a monovalent hydrocarbon group having 1-20 carbon atoms, or a monovalent hydrocarbon group having 2-20 carbon atoms which contains an etheric oxygen atom between carbon atoms.

R⁵¹: a monovalent organic group having an electron-withdrawing group. R³¹, R⁴¹, and R⁵¹ may be bonded to each other to form a divalent organic group or a trivalent organic group.

R^{E21} and R^{E22}: each independently a hydrogen atom or a monovalent organic group having 1-20 carbon atoms.

R⁶¹: a monovalent organic group having 1-20 carbon atoms.

R^{31F}: a group formed by fluorinating some or all of the hydrogen atoms or R³¹.

R^{41F}: a group formed by fluorinating some or all of the hydrogen atoms or R⁴¹.

R^{51F}: a group formed by fluorinating some or all of the hydrogen atoms or R⁵¹.

R^{61F}: a group formed by fluorinating some or all of the hydrogen atoms or R⁶¹.

R^{E21F} and R^{E22F}: each independently a fluorine atom or a group formed by fluorinating some or all of the hydrogen atoms of a monovalent organic group having 1-20 carbon atoms.

R¹¹ is a hydrogen atom or a monovalent organic group having 1-20 carbon atoms. When R¹¹ is not a hydrogen atom, the R¹¹ and R^{31F} may be bonded to each other to form a divalent organic group.

Furthermore, a scheme of a series of syntheses in which compound (1b) is used as a starting material is shown below.

A method for producing a nitrogen-containing fluorine-containing compound, the method including a step in which a compound represented by the following formula (1b) is reacted with a compound having an amino-protecting group to obtain a compound represented by the following formula (3b)-1 and
a step in which the compound represented by the following formula (3b)-1 is reacted with a compound represented by the following formula (4b) to obtain a compound represented by the following formula (5b)
or including a step in which a compound represented by the following formula (1b) is reacted with a compound represented by the following formula (4b) to obtain a compound represented by the following formula (3b)-2 and
a step in which the compound represented by the following formula (3b)-2 is reacted with a compound having an amino-protecting group to obtain a compound represented by the following formula (5b),
the method further including a step in which the compound represented by the following formula (5b) is fluorinated to obtain at least one of a compound represented by the following formula (6b)-1 and a compound represented by the following formula (6b)-2 and
a step in which at least one of the compound represented by the following formula (6b)-1 and the compound represented by the following formula (6b)-2 is subjected to deprotection and scission of the -O-E³¹- to obtain a compound represented by the following formula (7b)-1 and/or a compound represented by the following formula (7b)-2.

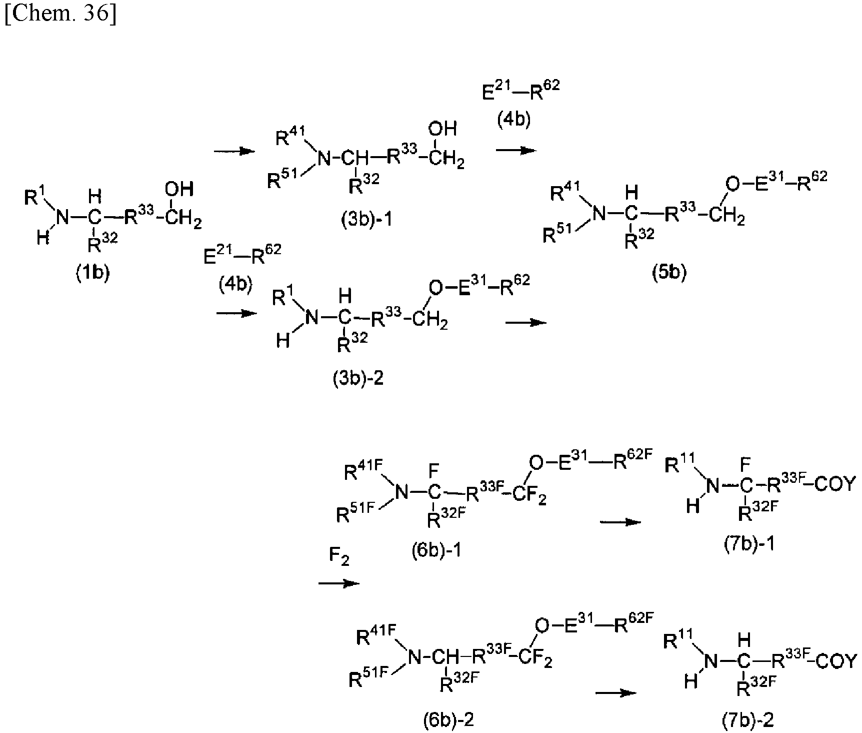

Definitions of the substituents in the formulae are as follows.

R¹ is a hydrogen atom, a monovalent hydrocarbon group having 1-20 carbon atoms, or a monovalent hydrocarbon group having 2-20 carbon atoms which contains an etheric oxygen atom between carbon atoms. When R¹ is not a hydrogen atom, the R¹ may be bonded to R³² to form a divalent organic group.

R³²: a hydrogen atom or a monovalent organic group having 1-19 carbon atoms.

R³³: a single bond or a divalent organic group having 1-10 carbon atoms, the total number of carbon atoms of R¹² and R³³ being 0-19.

R⁴¹: a monovalent organic group having an electron-withdrawing group, a monovalent hydrocarbon group having 1-20 carbon atoms, or a monovalent hydrocarbon group having 2-20 carbon atoms which contains an etheric oxygen atom between carbon atoms.

R⁵¹: a monovalent organic group having an electron-withdrawing group. R³², R⁴¹, and R⁵¹ may be bonded to each other to form a divalent organic group or a trivalent organic group.

E²¹: -COX or -SO₂X. X is a hydroxyl group or a halogen atom.

R⁶²: a monovalent organic group having 1-20 carbon atoms.

E³¹: -CO- or -SO₂-.

R^{32F}: a group formed by fluorinating some or all of the hydrogen atoms of R³².

R^{33F}: a group formed by fluorinating some or all of the hydrogen atoms of R³³, the total number of carbon atoms of R^{32F} and R^{33F} being 0-19.

R^{41F}: a group formed by fluorinating some or all of the hydrogen atoms of R⁴¹.

R^{51F}: a group formed by fluorinating some or all of the hydrogen atoms of R⁵¹.

R^{62F}: a group formed by fluorinating some or all of the hydrogen atoms of R⁶².

R¹¹: a hydrogen atom or a monovalent organic group having 1-20 carbon atoms. When R¹¹ is not a hydrogen atom, the R¹¹ and R^{32F} may be bonded to each other to form a divalent organic group.

Y: a hydroxyl group or a halogen atom.

### EXAMPLES

The present invention is explained in detail below by reference to Examples, but the present invention is not limited by the following Examples.

### Analytical Instruments

An NMR apparatus used for analyses in the Examples and Comparative Examples was JNM-ECZ400S (400 MHz), manufactured by JEOL Ltd. In ¹H NMR, tetramethylsilane was used for obtaining a reference value of 0 ppm. In ¹⁹F NMR, C₆F₆ was used for obtaining a reference value of -162 ppm. For GC-MS, use was made of 5977B, manufactured by Agilent Inc. For LC-MS, use was made of 6120, manufactured by Agilent Inc.

### [Example 1-1] Protection of Amino Group

The following compound (2)-1 (2.20 g), L-alanine (0.89 g), toluene (22 mL), and triethylamine (0.10 mL) were introduced in order into a 50-mL three-necked flask equipped with a stirrer, a Dimroth condenser (cooling-liquid temperature, 20°C), and a Dean-Stark distillation tube. The contents were heated and refluxed for 3 hours at an internal temperature of 110°C. After the starting material was ascertained by TLC to have disappeared, the solvent in the liquid reaction mixture was removed by distillation with a rotary evaporator. As a result, a white viscous solid separated out. Water and concentrated hydrochloric acid were added to the white solid, and the mixture was vigorously agitated to wash the solid. The white solid was taken out by filtration, and the solvent was removed therefrom by distillation at a reduced pressure to obtain 2.26 g of a white solid (the following compound (3a)-1-1) (yield, 78%). ¹H NMR (400 MHz, CDCl₃) δ 5.01 (q, 1H, J=8 Hz), 1.72 (d, 1H, 8 Hz). ¹⁹F NMR (376 MHz, CDCl₃) δ -133.1 (m, 2F), -141.8 (m, 2F).

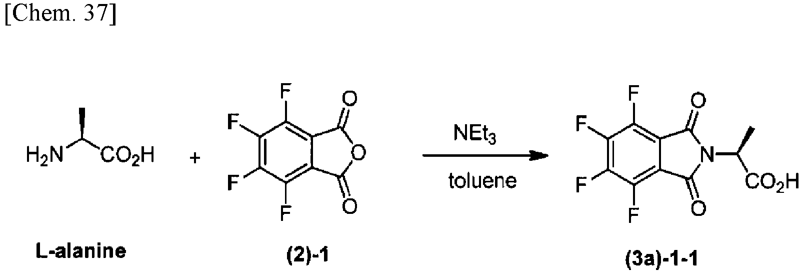

### [Example 1-2] Esterification

Into a 100-mL three-necked flask equipped with a stirrer were introduced the compound (3a)-1-1 (2.00 g) obtained in Example 1-1, dimethylaminopyridine (DMAP) (0.043 g), the following compound (4a)-1 (2.50 g), and methylene chloride (41 mL). The contents were cooled to 0°C. Thereto was added dicyclohexylcarbodiimide (DCC) (1.45 g). After 10 minutes, the temperature of the reaction solution was raised to 25°C and stirred for 3 hours. After the starting material was ascertained by TLC to have disappeared, the liquid reaction mixture, which contained a solid, was filtered to remove the solid. The filtrate was washed with saturated aqueous sodium bicarbonate solution and dried with sodium sulfate, and the solvent was thereafter removed by distillation at a reduced pressure to obtain 3.7 g of a crude product.

The crude product obtained in an amount of 3.7 g was purified by column chromatography (developing solvent: hexane/ethyl acetate (ethyl acetate, 5-100 vol%)) to obtain 3.0 g of a white solid (the following compound (5a)-1) (yield, 69%). ¹H NMR (400 MHz, CDCl₃) δ 4.96 (q, 1H, J=8 Hz), 4.48 (m, 2H), 2.46 (m, 2H), 1.70 (d, 3H, J=8 Hz). ¹⁹F NMR (376 MHz, CDCl₃) δ -81.1 (t, IF, J=10 Hz), -113.9 (m, 2F), -122.3 (bs, 2F), -123.2 (bs, 2F), -123.8 (bs, 2F), -126.5 (bs, 2F), -135.3 (m, 2F), -142.0 (m, 2F).

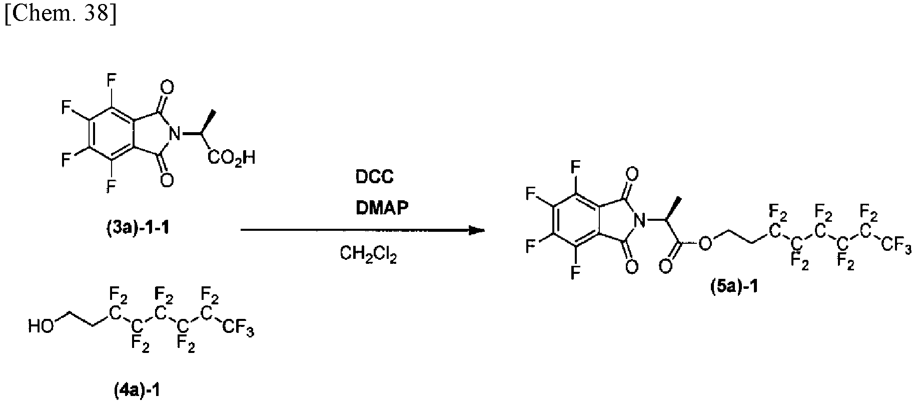

### [Example 1-3] Fluorination

Into a 500-mL autoclave made of nickel was introduced 1,1,2-trichloro-1,2,2-trifluoroethane (R-113, 250 g). The contents were stirred and kept at 25°C. Nitrogen gas was bubbled into the contents for 1 hour, and fluorine gas diluted to 20% with nitrogen gas was thereafter bubbled thereinto at a flow rate of 2.2 L/hr for 1 hour while keeping the internal pressure of the autoclave at ordinary pressure. While continuously bubbling the diluted fluorine gas at the same flow rate, a solution obtained by dissolving the compound (5a)-1 (4.99 g) obtained in Example 1-2 in R-113 (130 g) was added to the contents over 4.8 hours. Next, while continuously bubbling the diluted fluorine gas at the same flow rate and keeping the internal pressure of the autoclave at 0.1 MPaG, an R-113 solution (9 mL) having a benzene concentration of 0.01 g/mL was injected, the benzene injection port of the autoclave was closed, and the contents were continuously stirred for 0.3 hours while elevating the temperature from 25°C to 40°C. Subsequently, while keeping the internal temperature of the autoclave at 40°C, 6 mL of the benzene solution was injected, the benzene injection port of the autoclave was closed, and the contents were continuously stirred for 0.3 hours. Furthermore, the same operation was repeated once. The total benzene injection amount was 0.21 g and the total R-113 injection amount was 21 mL. While continuously bubbling the diluted fluorine gas at the same flow rate, stirring was continued for further 1 hour. Next, the internal pressure of the reactor was adjusted to ordinary pressure, and nitrogen gas was bubbled for 1 hour before the liquid reaction mixture in the autoclave was taken out. The solvent was removed by distillation at a reduced pressure to obtain 1.52 g of a colorless liquid crude product (the following compound (6a)-1-1 and compound (6a)-1-2) (mass yield, 83%; ¹⁹F NMR quantitative yield, 42%). In an examination of this crude product by GC-MS (ionization method: EI), characteristic fragment peaks were observed at 460.10 and 432.09.

The crude product was reacted at 25°C with a mixed solvent which was a 75/25 mixture of water and acetonitrile, the amount of the mixed solvent being 3,000 times by volume the amount of the crude product, and the resultant solution was examined by LC-MS. As a result, a peak was observed at M/Z=476 (M-H), the peak being thought to be attributable to compound (6a)-1-3 obtained by the hydrolysis of the compound (6a)-1-1 and the compound (6a)-1-2. Compound (6a)-1-1 and Compound (6a)-1-2: ¹⁹F NMR (376 MHz, CDCl₃) δ -72.9 (m, 3F), -81.0 (t, 3F, J=10 Hz), -86.7 (m, 2F), -121.0--123.0 (m, 15F), -125.0--128.0 (m, 9F), -130.0--134.0 (m, 4F), -143.3 (s, IF), -188.2 (m, 2F). Compound (6a)-1-1: molecular weight, 460.10. Compound (6a)-1-2: molecular weight, 432.09. Compound (6a)-1-3: molecular weight, 477.11.

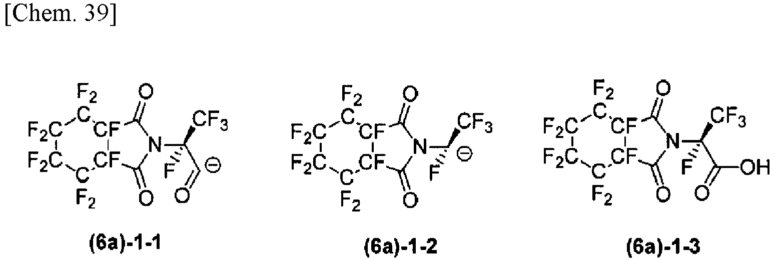

### [Example 2-1] Esterification

Into a 100-mL two-necked flask equipped with a stirrer were introduced the compound (3a)-1-1 (2.827 g) obtained in Example 1-1, DMAP (0.089 g), the following compound (4a)-2 (4.80 g), and methylene chloride (22 mL). The contents were cooled to 0°C. Thereto was added 1-(3-dimethylaminopropyl)-3-ethylcarbodiimide hydrochloride (EDC) (2.82 g). After 10 minutes, the temperature of the reaction solution was raised to 25°C and stirred for 16 hours. After the starting material was ascertained by TLC to have disappeared, saturated aqueous sodium bicarbonate solution was added and the resultant mixture was extracted with methylene chloride. The obtained organic layer was dried with sodium sulfate, and the solvent was thereafter removed by distillation at a reduced pressure to obtain 6.1 g of a crude product.

The obtained crude product was purified by column chromatography (developing solvent: hexane/ethyl acetate (ethyl acetate, 5-100 vol%)) to obtain 5.4 g of a white solid (the following compound (5a)-2) (yield, 94%). ¹H NMR (400 MHz, CDCl₃) δ 1.718 (d, 3H, J=7 Hz), 4.71 (m, 2H), 5.03 (q, 1H, J=7 Hz). ¹⁹F NMR (376 MHz, CDCl₃) δ -141.2 (m, 2F), -134.6 (m, 2F), -134.3 (m, 1F), -129.7 (m, 2F), -82.7 (d, 3F, J=32 Hz), -81.8 (m, 2F), -81.2 (m, 3F).

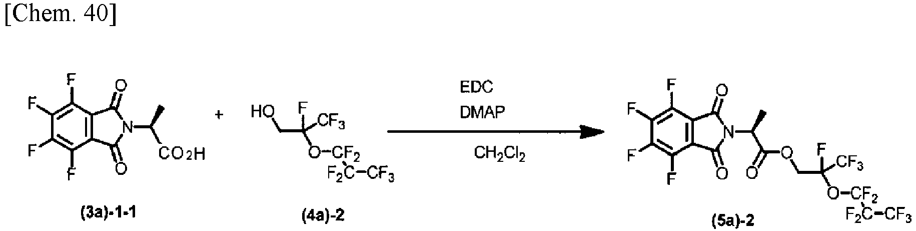

### [Example 2-2] Fluorination

ClCF₂CFClCF₂OCF₂CF₂Cl (CFE-419) (117 g) was introduced into a 200-mL autoclave made of nickel. The contents were stirred and kept at 25°C. Nitrogen gas was bubbled into the contents for 1 hour, and fluorine gas diluted to 20% with nitrogen gas was thereafter bubbled thereinto at a flow rate of 1.8 L/hr for 1 hour while keeping the internal pressure of the autoclave at ordinary pressure. While continuously bubbling the diluted fluorine gas at the same flow rate, a solution obtained by dissolving the compound (5a)-2 (0.7 g) obtained in Example 2-1 in CFE-419 (66 g) was added to the contents over 2 hours.
Furthermore, while bubbling the diluted fluorine gas at the same flow rate, stirring was continued for 20 minutes. Next, CFE-419 (20 g) was added and nitrogen gas was bubbled into the contents for 1 hour. Thereafter, the liquid reaction mixture in the autoclave was taken out, and the solvent was removed by distillation at a reduced pressure to obtain 0.74 g of a colorless liquid crude product (mass yield, 77%; compound (6a)-1-4, ¹⁹F NMR quantitative yield, 30%; compound (6a)-2-1, ¹H NMR quantitative yield, 29%). Compound (6a)-1-4 ¹⁹F NMR (376 MHz, CDCl₃) δ -72.9 (m, 3F), -78.0--83.0 (m, 8F), -86.2 (m, 2F), -121.0₋-125.0 (m, 4F), -129.3 (m, 2F), -130.0--134.0 (m, 4F), -142.6 (m, 1F), -144.8 (m, 1F), -188.2 (m, 2F). Compound (6a)-2-1 ¹H NMR (400 MHz, CDCl₃) δ 5.50 (bs, 1H); ¹⁹F NMR (376 MHz, CDCl₃) δ -72.9 (m, 3F), -78.0--83.0 (m, 8F), -86.2 (m, 2F), -121.0--125.0 (m, 4F), -129.3 (m, 2F), -130.0--134.0 (m, 4F), -144.8 (m, 1F), -188.2 (m, 2F). Compound (5a)-2: molecular weight, 589.21. Compound (6a)-1-4: molecular weight, 811.15. Compound (6a)-2-1: molecular weight, 793.16.

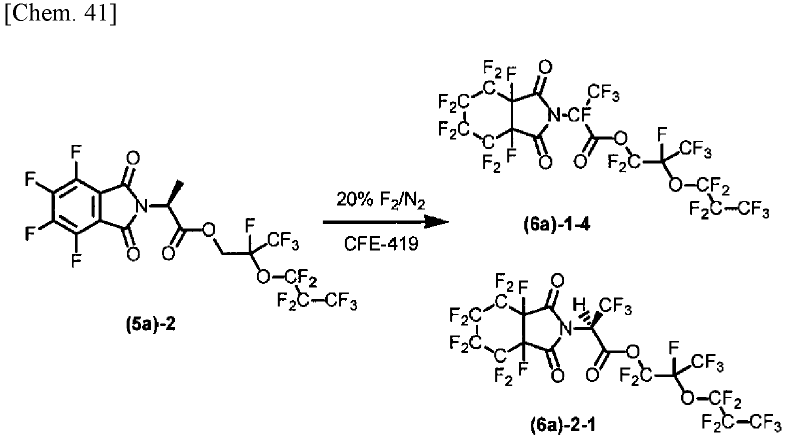

### [Example 3-1] Protection of Amino Group

The same procedure as in Example 1-1 was conducted except that L-valine was used in place of the L-alanine as a starting material, thereby obtaining the following compound (3a)-1-2 (yield, 91%). ¹H NMR (400 MHz, CDCl₃) δ 0.910 (d, 3H, J=7 Hz), δ 1.149 (d, 3H, J=7 Hz), δ 2.720 (m, 1H), 4.585 (d, 1H, J=8 Hz). ¹⁹F NMR (376 MHz, CDCl₃) δ -134.6 (m, 2F), -141.4 (m, 2F).

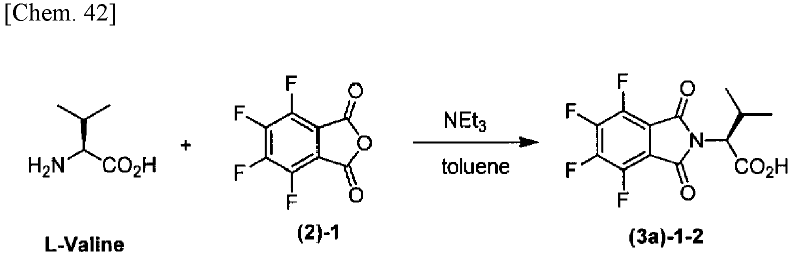

### [Example 3-2] Esterification

Into a 100-mL two-necked flask equipped with a stirrer were introduced the compound (3a)-1-2 (1.95 g) obtained in Example 3-1, DMAP (0.063 g), the following compound (4a)-2 (2.85 g), and methylene chloride (30 mL). The contents were cooled to 0°C. Thereto was added EDC (1.79 g). After 10 minutes, the temperature of the reaction solution was raised to 25°C and stirred for 2.5 hours. After the starting material was ascertained by TLC to have disappeared, saturated aqueous sodium bicarbonate solution was added and the resultant mixture was extracted with methylene chloride. The obtained organic layer was washed with saturated aqueous sodium bicarbonate solution and brine and dried with sodium sulfate, and the solvent was thereafter removed by distillation at a reduced pressure to obtain a crude product. The obtained crude product was purified by column chromatography (developing solvent: hexane/ethyl acetate (4/1)) to obtain 3.2 g of a white solid (the following compound (5a)-3) (yield, 95%). ¹H NMR (400 MHz, CDCl₃) δ 0.90 (d, 3H, J=7 Hz), 1.13 (d, 3H, J=7 Hz), 2.70 (m, 1H), 4.6 (d, 1H, J=8 Hz), 4.66 (m, 2H). ¹⁹F NMR (376 MHz, CDCl₃) δ -81.2 (m, 3F), -82.0 (m, 1F), -82.4 (m, 2F), -82.8 (m, 2F), -129.6 (m, 2F), -134.2 (m, 1F), -134.6 (m, 2F), -141.1 (m, 2F).

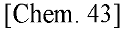

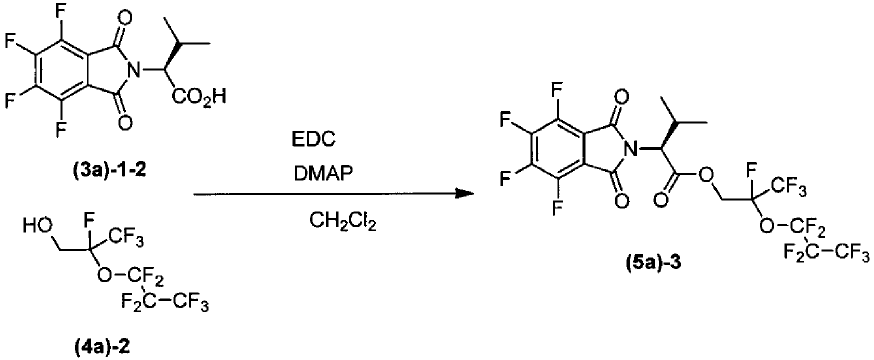

### [Example 3-3] Fluorination

CFE-419 (117 g) was introduced into a 200-mL autoclave made of nickel. The contents were stirred and kept at 25°C. Nitrogen gas was bubbled into the contents for 1 hour, and fluorine gas diluted to 20% with nitrogen gas was thereafter bubbled thereinto at a flow rate of 5.4 L/hr for 1 hour while keeping the internal pressure of the autoclave at ordinary pressure. While continuously bubbling the diluted fluorine gas at the same flow rate, a solution obtained by dissolving the compound (5a)-3 (2.0 g) obtained in Example 3-2 in CFE-419 (65 g) was added to the contents over 2 hours. Furthermore, while bubbling the diluted fluorine gas at the same flow rate, stirring was continued for 20 minutes. Next, CFE-419 (20 g) was added and nitrogen gas was bubbled into the contents for 1 hour. Thereafter, the liquid reaction mixture in the autoclave was taken out, and the solvent was removed by distillation at a reduced pressure to obtain 2.50 g of a colorless liquid crude product (mass yield, 86%; compound (6a)-1-5, ¹⁹F NMR quantitative yield, 49%; compound (6a)-2-2-1, ¹H NMR quantitative yield, 18%). Compound (6a)-1-5 ¹⁹F NMR (376 MHz, CDCl₃) δ -73.0--69.3 (5F), -79.3-81.2 (3F), -83.6-81.1 (6F), -89.5-85.5 (2F), -127.8--118.7 (4F), -130.30 (2F), -139.4--130.7 (4F), -147.0--144.7 (2F), -175.50 (1F), -191.0--187.3 (2F). Compound (6a)-2-2-1 ¹H NMR (400 MHz, CDCl₃) δ 5.35 (m, 1H); ¹⁹F NMR (376 MHz, CDCl₃) δ -73.0--69.3 (5F), -79.3-81.2 (3F), -83.6-81.1 (6F), -89.5-85.5 (2F), -127.8--118.7 (4F), -130.30 (2F), -139.4--130.7 (4F), -147.0--144.7 (2F), -175.50 (1F), -191.0--187.3 (2F). Compound (5a)-3: molecular weight, 617.27. Compound (6a)-1-5: molecular weight, 911.16. Compound (6a)-2-2-1: molecular weight, 893.17.

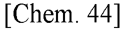

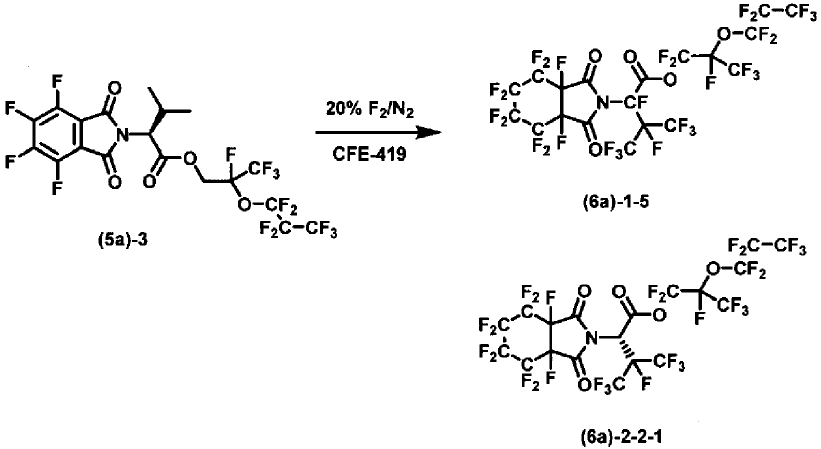

### [Example 4-1] Protection of Amino Group

The same procedure as in Example 1-1 was conducted except that hexafluoro-L-valine was used in place of the L-alanine as a starting material, thereby synthesizing the following compound (3a)-1-3 (yield, 83%). ¹H NMR (400 MHz, CDCl₃) δ 4.38 (m, 1H), 5.32 (d, 1H, J=6 Hz). ¹⁹F NMR (376 MHz, CDCl₃) δ -63.2 (m, 3F), -65.1 (m, 3F), -133.3 (m, 2F), -140.3 (m, 2F).

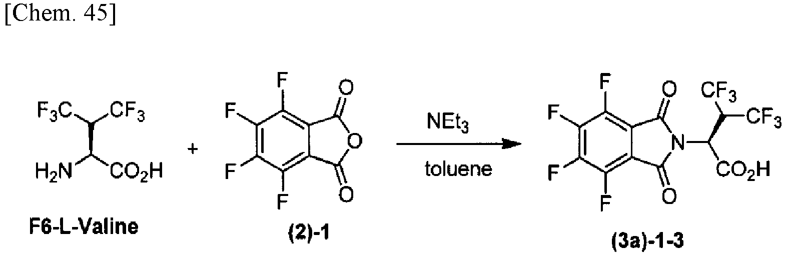

### [Example 4-2] Esterification

The same procedure as in Example 3-2 was conducted except that the compound (3a)-1-3 obtained in Example 4-1 was used in place of the compound (3a)-1-2 as a starting material, thereby obtaining the following (5a)-4 (yield, 54%). ¹H NMR (400 MHz, CDCl₃) δ 4.35 (m, 1H), 4.74 (m, 2H), 5.36 (m, 1H). ¹⁹F NMR (376 MHz, CDCl₃) δ -63.3 (m, 3F), -65.2 (m, 3F), -81.2 (m, 3F), -82.0 (m, 1F), -82.2 (m, 2F), -82.5 (m, 2F), -129.6 (m, 2F), -132.6 (m, 2F), -134.6 (m, 1F), -139.3 (m, 2F).

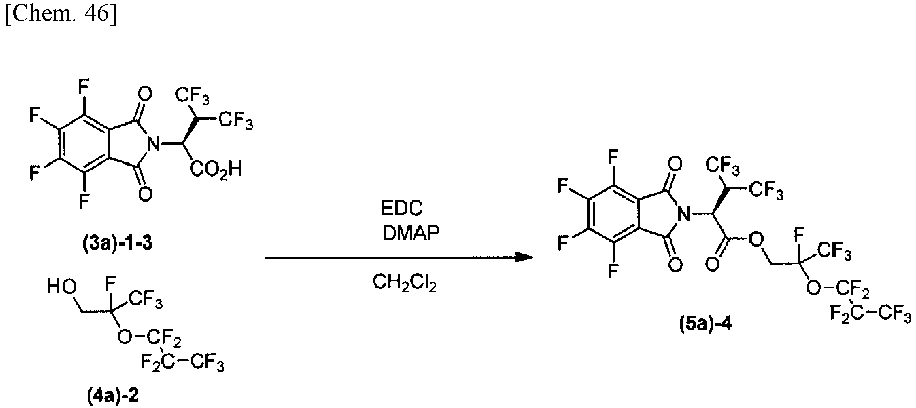

### [Example 4-3] Fluorination

CFE-419 (117 g) was introduced into a 200-mL autoclave made of nickel. The contents were stirred and kept at 25°C. Nitrogen gas was bubbled into the contents for 1 hour, and fluorine gas diluted to 20% with nitrogen gas was thereafter bubbled thereinto at a flow rate of 1.1 L/hr for 1 hour while keeping the internal pressure of the autoclave at ordinary pressure. While continuously bubbling the diluted fluorine gas at the same flow rate, a solution obtained by dissolving the compound (5a)-4 (0.60 g) obtained in Example 4-2 in CFE-419 (33 g) was added to the contents over 2 hours. Furthermore, while bubbling the diluted fluorine gas at the same flow rate, stirring was continued for 20 minutes. Next, CFE-419 (20 g) was added and nitrogen gas was bubbled into the contents for 1 hour. Thereafter, the liquid reaction mixture in the autoclave was taken out, and the solvent was removed by distillation at a reduced pressure to obtain 0.68 g of a colorless liquid crude product (mass yield, 91%; compound (6a)-1-6, ¹⁹F NMR quantitative yield, 31%; compound (6a)-2-3-1, ¹H NMR quantitative yield, 34%). Compound (6a)-1-6 ¹⁹F NMR (376 MHz, CDCl₃) δ -73.0--69.3 (5F), -79.3-81.2 (3F), -83.6-81.1 (6F), -89.5-85.5 (2F), -127.8--118.7 (4F), -130.30 (2F), -139.4--130.7 (4F), -147.0--144.7 (2F), -175.50 (1F), -191.0--187.3 (2F). Compound (6a)-2-3-1 ¹H NMR (400 MHz, CDCl₃) δ 5.35 (m, 1H); ¹⁹F NMR (376 MHz, CDCl₃) δ -73.0--69.3 (5F), -79.3-81.2 (3F), -83.6-81.1 (6F), -89.5-85.5 (2F), -127.8--118.7 (4F), -130.30 (2F), -139.4--130.7 (4F), -147.0--144.7 (2F), -175.50 (1F), -191.0--187.3 (2F). Compound (5a)-4: molecular weight, 725.2. Compound (6a)-1-6: molecular weight, 911.16. Compound (6a)-2-3-1: molecular weight, 893.17.

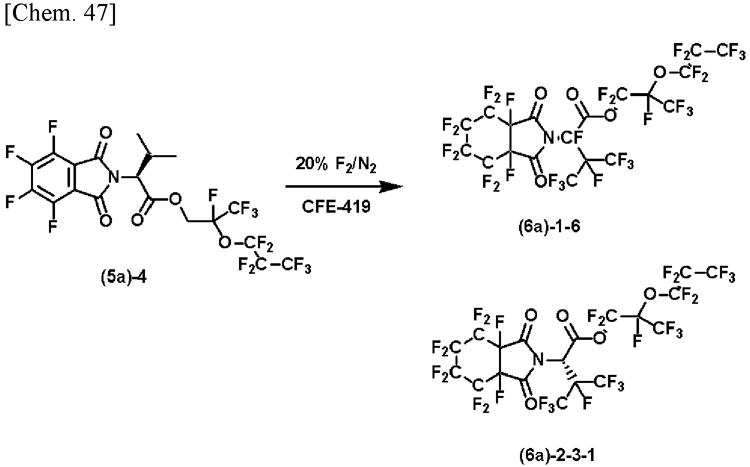

### [Example 5-1] Protection of Amino Group

The same procedure as in Example 1-1 was conducted except that 1-aminopropanol (0.76 g) was used in place of the L-alanine as a starting material, thereby obtaining the following compound (3b)-1-1. The crude product thus obtained was purified by silica gel column chromatography (developing solvent, hexane/ethyl acetate) to obtain 1.87 g of a white solid (yield, 67%). ¹H NMR (400 MHz, CDCl₃) δ 3.85 (t, 2H, J=6 Hz), 3.85 (t, 2H, J=6 Hz), 1.90 (m, 3H). ¹⁹F NMR (376 MHz, CDCl₃) δ -135.6 (m, 2F), -142.3 (m, 2F).

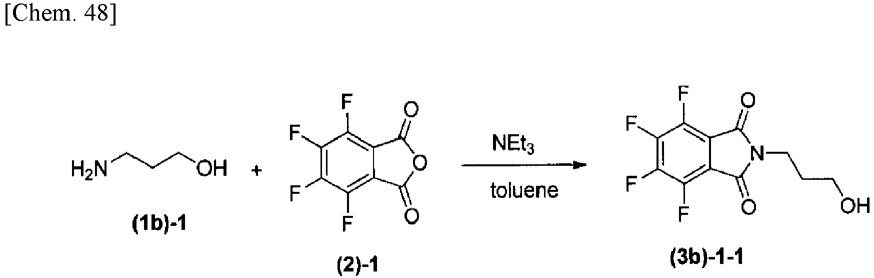

### [Example 5-2]

Into a 100-mL three-necked flask equipped with a stirrer were introduced the compound (3b)-1-1 (1.01 g) obtained in Example 5-1 and dichloropentafluoropropane (trade name AK-225, manufactured by AGC Inc.; 20 mL). The contents were cooled to 0°C, and triethylamine (0.79 g) was then added thereto. Compound (4b)-1 (1.37 g) was gradually added thereto, and the resultant mixture was stirred at 25°C overnight. Saturated aqueous sodium bicarbonate solution was added to the liquid reaction mixture, and the resultant mixture was subjected to liquid separation. The aqueous phase was extracted with AK-225 (10 mL). This extract was added to the organic phase and the mixture was washed with saturated aqueous sodium chloride solution. The solvent was removed by distillation, and the obtained crude product was purified by silica gel column chromatography (developing solvent: hexane/ethyl acetate) to obtain 1.92 g of a white solid (compound (5b)-1) (yield, 90%). ¹H NMR (400 MHz, CDCl₃) δ 4.43 (m, 2H), 3.80 (t, 2H, J=7 Hz), 2.13 (m, 3H). ¹⁹F NMR (376 MHz, CDCl₃) δ -80.3 (d, IF, J=147 Hz), -81.5 (s, 3F), -82.3 (s, 3F), -86.6 (d, IF, J=147 Hz), -129.9 (m, 2F), -132.3 (m, 1F), -135.3 (m, 2F), -142.0 (m, 2F).

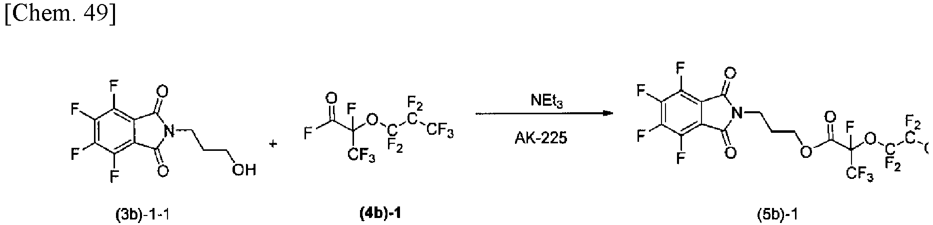

### [Example 5-3] Fluorination

CFE-419 (117 g) was introduced into a 200-mL autoclave made of nickel. The contents were stirred and kept at 25°C. Nitrogen gas was bubbled into the contents for 1 hour, and fluorine gas diluted to 20% with nitrogen gas was thereafter bubbled thereinto at a flow rate of 1.8 L/hr for 1 hour while keeping the internal pressure of the autoclave at ordinary pressure. While continuously bubbling the diluted fluorine gas at the same flow rate, a solution obtained by dissolving the compound (5b)-1 (0.70 g) obtained in Example 5-2 in CFE-419 (67 g) was added to the contents over 2 hours. Furthermore, while bubbling the diluted fluorine gas at the same flow rate, stirring was continued for 20 minutes. Next, CFE-419 (20 g) was added and nitrogen gas was bubbled into the contents for 1 hour. Thereafter, the liquid reaction mixture in the autoclave was taken out, and the solvent was removed by distillation at a reduced pressure to obtain 0.80 g of a colorless liquid crude product (mass yield, 83%; compound (6b)-1-1, ¹⁹F NMR quantitative yield, 47%). ¹⁹F NMR (376 MHz, CDCl₃) δ -79.0 (m, 1F), -80.7-82.1 (m, 6F), -85.9-86.3 (m, 3F), -97.5 (m, 2F), -121.5-124.3 (m, 4F), -125.9 (m, 2F), -129.3 (m, 3F), -131.9--138.0 (m, 4F), -188.3 (s, 2F). Compound (5b)-1: molecular weight, 589.21. Compound (6b)-1-1: molecular weight, 811.15.

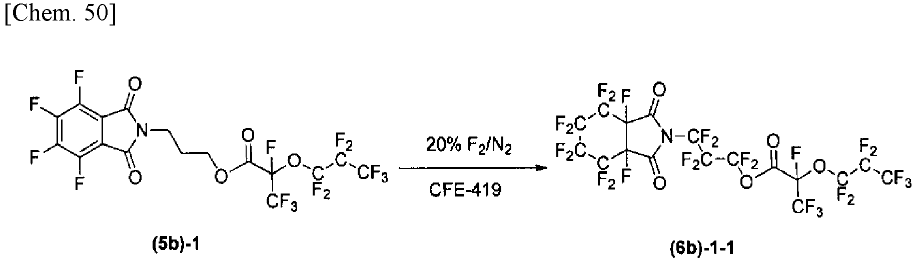

### [Example 6-1] Protection of Amino Group

Succinic anhydride (5.30 g) and L-alanine (4.45 g) were introduced into a 50-mL three-necked flask equipped with a stirrer and a Dimroth condenser (cooling-liquid temperature, 20°C). The contents were heated at 140°C for 6.5 hours and then cooled. To the liquid reaction mixture were added 20 mL of water and 20 mL of ethyl acetate. The resultant mixture was subjected to liquid separation, and the aqueous phase was extracted with ethyl acetate (2×10 mL). The extract was washed with an aqueous sodium chloride solution and then dried with sodium sulfate. The solvent was removed from the organic phase by distillation, and 54 mL of toluene was added to the solid thus obtained. This mixture was heated and refluxed and, as a result, the solid dissolved. The solution was cooled to 25°C, and the obtained solid was taken out by filtration to obtain 1.69 g of phthaloyl-L-alanine (yield, 20%). ¹H NMR (400 MHz, CDCl₃) δ 4.61 (q, 1H, J=7 Hz), 2.66 (m, 4H), 1.36 (d, 1H, 8 Hz).

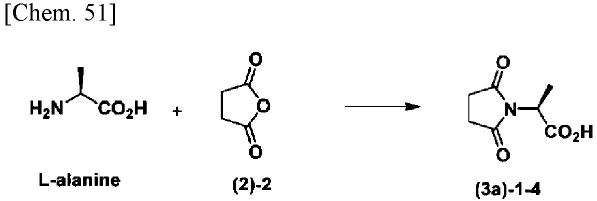

### [Example 6-2] Esterification

The same procedure as in Example 3-2 was conducted except that the compound (3a)-1-4 obtained in Example 6-1 was used in place of the compound (3a)-1-2 as a starting material, thereby obtaining the following compound (5a)-5 (yield, 73%). ¹H NMR (400 MHz, CDCl₃) δ 4.90 (q, 1H, J=7 Hz), 4.65 (m, 2H), 2.75 (m, 4H), 1.58 (d, 3H, J=7 Hz). ¹⁹F NMR (376 MHz, CDCl₃) δ -81--84 (m, 8F), -130.0 (m, 2F), -134.5 (m, 1F).

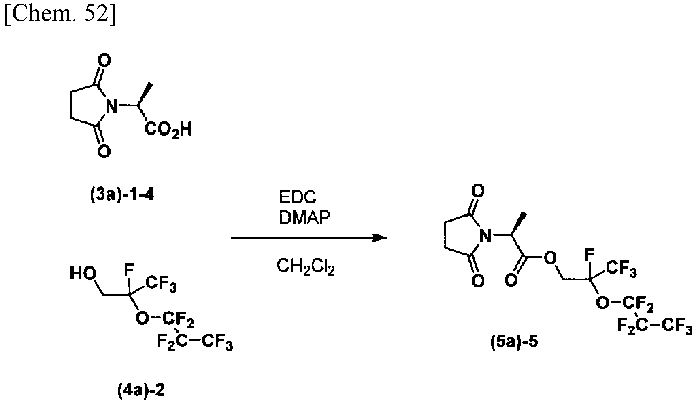

### [Example 6-3] Fluorination

CFE-419 (117 g) was introduced into a 200-mL autoclave made of nickel. The contents were stirred and kept at 25°C. Nitrogen gas was bubbled into the contents for 1 hour, and fluorine gas diluted to 20% with nitrogen gas was thereafter bubbled thereinto at a flow rate of 3.6 L/hr for 1 hour while keeping the internal pressure of the autoclave at ordinary pressure. While continuously bubbling the diluted fluorine gas at the same flow rate, a solution obtained by dissolving the compound (5a)-5 (1.0 g) obtained in Example 6-2 in CFE-419 (66 g) was added to the contents over 2 hours. Furthermore, while bubbling the diluted fluorine gas at the same flow rate, stirring was continued for 20 minutes. Next, CFE-419 (12 g) was added and nitrogen gas was bubbled into the contents for 1 hour. Thereafter, the liquid reaction mixture in the autoclave was taken out, and the solvent was removed by distillation at a reduced pressure to obtain 0.68 g of a colorless liquid crude product (mass yield, 73%; compound (6a)-1-6). ¹⁹F NMR (376 MHz, CDCl₃) δ -73.0 (3F), -78--82 (10F), -105--106 (4F), -129 (2F), -132 (1F), -144 (1F).

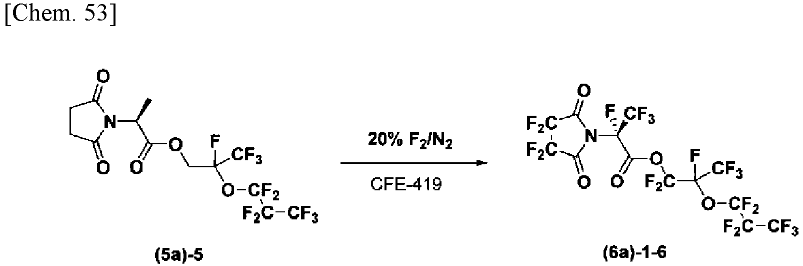

### [Example 7-1] Esterification

The same procedure as in Example 3-2 was conducted except that commercial compound (3a)-1-5 was used in place of the compound (3a)-1-2 as a starting material, thereby obtaining the following compound (5a)-6 (yield, 97%). ¹H NMR (400 MHz, CDCl₃) δ 7.89 (m, 2H), 7.75 (m, 2H), 5.10 (q, 1H, J=7 Hz), 4.68 (m, 2H), 1.73 (d, 3H, J=7 Hz). ¹⁹F NMR (376 MHz, CDCl₃) δ -81--84 (m, 8F), -130.0 (m, 2F), -134.4 (m, 1F).

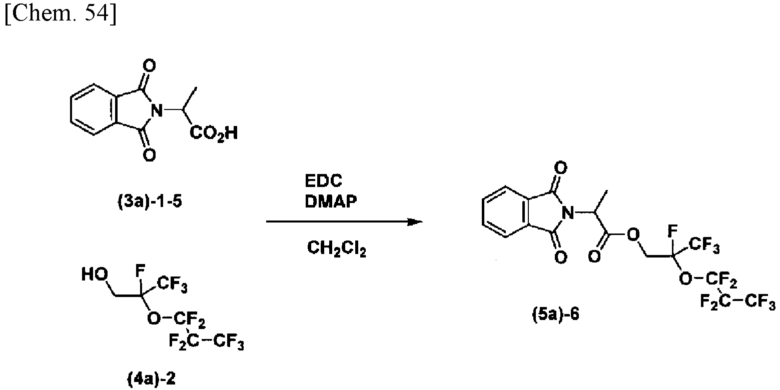

### [Example 7-2] Fluorination

CFE-419 (117 g) was introduced into a 200-mL autoclave made of nickel. The contents were stirred and kept at 25°C. Nitrogen gas was bubbled into the contents for 1 hour, and fluorine gas diluted to 20% with nitrogen gas was thereafter bubbled thereinto at a flow rate of 4.3 L/hr for 1 hour while keeping the internal pressure of the autoclave at ordinary pressure. While continuously bubbling the diluted fluorine gas at the same flow rate, a solution obtained by dissolving the compound (5a)-6 (1.0 g) obtained in Example 7-1 in CFE-419 (53 g) was added to the contents over 2 hours. Furthermore, while bubbling the diluted fluorine gas at the same flow rate, stirring was continued for 20 minutes. Next, CFE-419 (12 g) was added and nitrogen gas was bubbled into the contents for 1 hour. Thereafter, the liquid reaction mixture in the autoclave was taken out, and the solvent was removed by distillation at a reduced pressure to obtain 0.68 g of a colorless liquid crude product (mass yield, 78%; compound (6a)-1-4, ¹⁹F NMR quantitative yield, 26%; compound (6a)-2-1-1, ¹H NMR quantitative yield, 18%). Compound (6a)-1-4 ¹⁹F NMR (376 MHz, CDCl₃) δ -72.9 (m, 3F), -78.0--83.0 (m, 8F), -86.2 (m, 2F), -121.0₋-125.0 (m, 4F), -129.3 (m, 2F), -130.0--134.0 (m, 4F), -142.6 (m, 1F), -144.8 (m, 1F), -188.2 (m, 2F). Compound (6a)-2-1-1 ¹H NMR (400 MHz, CDCl₃) δ 5.50 (bs, 1H); ¹⁹F NMR (376 MHz, CDCl₃) δ -72.9 (m, 3F), -78.0--83.0 (m, 8F), -86.2 (m, 2F), -121.0₋-125.0 (m, 4F), -129.3 (m, 2F), -130.0- -134.0 (m, 4F), -144.8 (m, 1F), -188.2 (m, 2F).

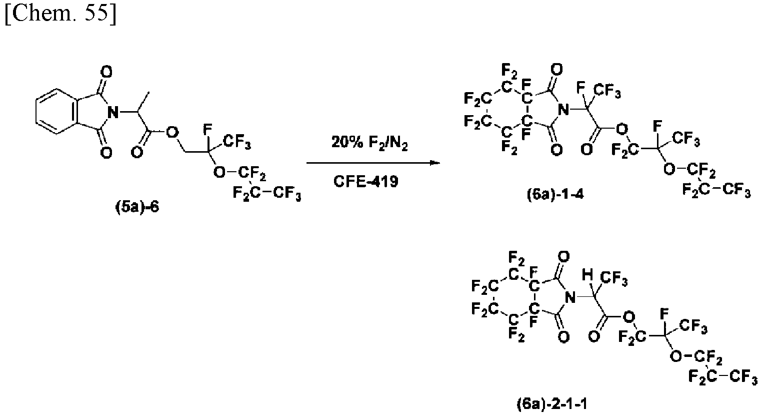

### INDUSTRIAL APPLICABILITY

Nitrogen-containing fluorine-containing compounds are useful compounds for various purposes including solvents, surfactants, and medicines. According to the present invention, nitrogen-containing fluorine-containing compounds which have hitherto been difficult to synthesize because of low fluorination yields are obtained in high yields.

While the invention has been described in detail and with reference to specific embodiments thereof, it will be apparent to one skilled in the art that various changes and modifications can be made therein without departing from the spirit and scope thereof. This application is based on a Japanese patent application filed on August 20, 2019 (Application No. 2019-150741), the entire contents thereof being incorporated herein by reference.

## Claims

1. A method for producing a nitrogen-containing fluorine-containing compound, the method comprising a step in which a compound represented by the following formula (5) is fluorinated to obtain a compound represented by the following formula (6): 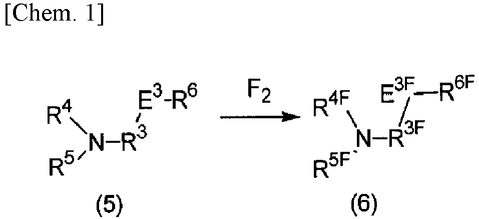 wherein definitions of substituents in the formulae are as follows:
R³: a single bond or a divalent organic group having 1-20 carbon atoms;
R⁴ and R⁵ are each independently a monovalent organic group having an electron-withdrawing group, a monovalent hydrocarbon group having 1-20 carbon atoms, or a monovalent hydrocarbon group having 2-20 carbon atoms which contains an etheric oxygen atom between carbon atoms, at least one of R⁴ and R⁵ being a monovalent organic group having an electron-withdrawing group, where R³, R⁴, and R⁵ may be bonded to each other to form a divalent organic group or a trivalent organic group;
R⁶: a monovalent organic group having 1-20 carbon atoms;
E³: a divalent organic group formed by a reaction between the following E¹ and E²;
E¹: -COX, -SO₂X, or -C(R^{E11})(R^{E12})OH, where X is a hydroxyl group or a halogen atom, and R^{E11} and R^{E12} are each independently a hydrogen atom or a monovalent organic group having 1-10 carbon atoms;
E²: -C(R^{E21})(R^{E22})OH, -COX, or -SO₂X, where R^{E21} and R^{E22} are each independently a hydrogen atom or a monovalent organic group having 1-20 carbon atoms, and X is a hydroxyl group or a halogen atom;
R^{3F}: a group formed by fluorinating some or all of hydrogen atoms of R³;
R^{4F}: a group formed by fluorinating some or all of hydrogen atoms of R⁴;
R^{5F}: a group formed by fluorinating some or all of hydrogen atoms of R⁵;
R^{6F}: a group formed by fluorinating some or all of hydrogen atoms of R⁶;
E^{3F}: a group formed by fluorinating some or all of hydrogen atoms of E³.

2. The production method according to claim 1, further comprising a step in which a compound represented by the following formula (3)-1 is reacted with a compound represented by the following formula (4) to obtain the compound represented by the following formula (5) or
a step in which a compound represented by the following formula (3)-2 is reacted with a compound having an amino-protecting group to obtain the compound represented by the following formula (5): 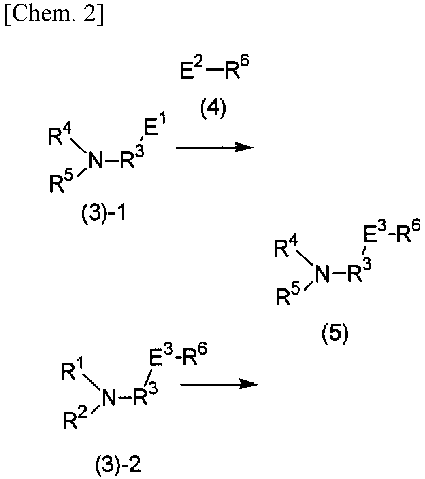
wherein definitions of substituents in the formulae are as follows:
R¹ and R² are each independently a hydrogen atom, a monovalent hydrocarbon group having 1-20 carbon atoms, or a monovalent hydrocarbon group having 2-20 carbon atoms which contains an etheric oxygen atom between carbon atoms, at least one of R¹ and R² being a hydrogen atom, and in the case where R¹ or R² is not a hydrogen atom, then the R¹ or R² may be bonded to R³ to form a divalent organic group;
R³, R⁴, R⁵, R⁶, E¹, E², and E³: the same as in claim 1.

3. The production method according to claim 2, further comprising a step in which a compound represented by the following formula (1) is reacted with a compound having an amino-protecting group to obtain the compound represented by the following formula (3)-1 or
a step in which a compound represented by the following formula (1) is reacted with a compound represented by the following formula (4) to obtain the compound represented by the following formula (3)-2: 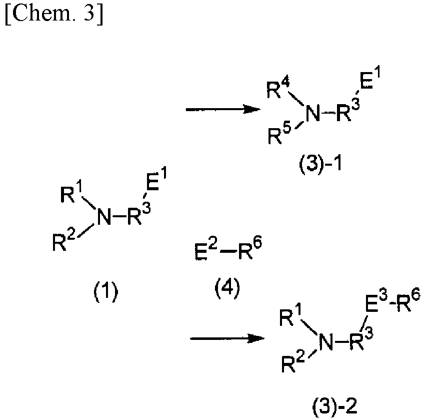
wherein definitions of substituents in the formulae are as follows:
R¹, R², R³, R⁴, R⁵, R⁶, E¹, E², and E³: the same as in claim 1 or 2.

4. The production method according to any one of claims 1 to 3, further comprising a step in which the compound represented by the following formula (6) is deprotected and caused to undergo scission of the linking group E^{3F}, thereby obtaining a compound represented by the following formula (7): 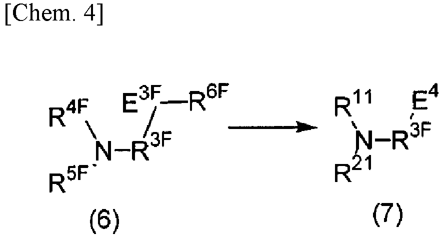 wherein definitions of substituents in the formulae are as follows:
R¹¹ and R²¹ are each independently a hydrogen atom or a monovalent organic group having 1-20 carbon atoms, at least one of R¹¹ and R²¹ being a hydrogen atom, and in the case where R¹¹ or R²¹ is not a hydrogen atom, then the R¹¹ or R²¹ may be bonded to R^{3F} to form a divalent organic group;
E⁴: -COX or -SO₂X, where X is a hydroxyl group or a halogen atom;
R^{3F}, R^{4F}, R^{5F}, R^{6F}, and E^{3F}: the same as in claim 1.

5. A compound represented by the following formula (5): 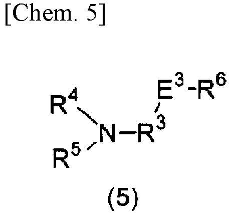 wherein definitions of substituents in the formula are as follows:
R³: a single bond or a divalent organic group having 1-20 carbon atoms;
R⁴ and R⁵ are each independently a monovalent organic group having an electron-withdrawing group, a monovalent hydrocarbon group having 1-20 carbon atoms, or a monovalent hydrocarbon group having 2-20 carbon atoms which contains an etheric oxygen atom between carbon atoms, at least one of R⁴ and R⁵ being a monovalent organic group having an electron-withdrawing group, where R³, R⁴, and R⁵ may be bonded to each other to form a divalent organic group or a trivalent organic group;
R⁶: a monovalent organic group having 1-20 carbon atoms;
E³: a divalent organic group formed by a reaction between the following E¹ and E²;
E¹: -COX, -SO₂X, or -C(R^{E11})(R^{E12})OH, where X is a hydroxyl group or a halogen atom, R^{E11} and R^{E12} are each independently a hydrogen atom or a monovalent organic group having 1-10 carbon atoms;
E²: -C(R^{E21})(R^{E22})OH, -COX, or -SO₂X, where R^{E21} and R^{E22} are each independently a hydrogen atom or a monovalent organic group having 1-20 carbon atoms, and X is a hydroxyl group or a halogen atom.

6. A compound represented by the following formula (5a) or a compound represented by the following formula (5b): 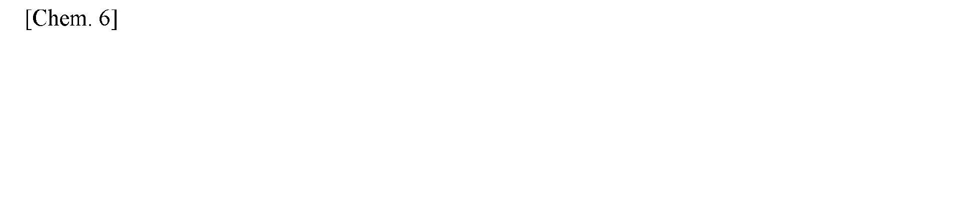 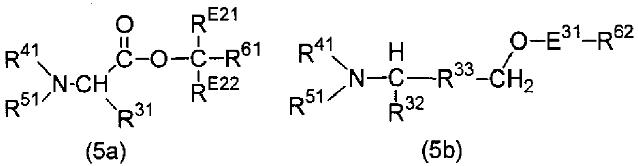 wherein definitions of substituents in the formulae are as follows:
R³¹: a hydrogen atom or a monovalent organic group having 1-19 carbon atoms;
R³²: a hydrogen atom or a monovalent organic group having 1-19 carbon atoms;
R³³: a single bond or a divalent organic group having 1-10 carbon atoms;
R⁴¹: a monovalent organic group having an electron-withdrawing group, a monovalent hydrocarbon group having 1-20 carbon atoms, or a monovalent hydrocarbon group having 2-20 carbon atoms which contains an etheric oxygen atom between carbon atoms;
R⁵¹: a monovalent organic group having an electron-withdrawing group, where R³¹, R⁴¹, and R⁵¹ may be bonded to each other to form a divalent organic group or a trivalent organic group, and R³², R⁴¹, and R⁵¹ may be bonded to each other to form a divalent organic group or a trivalent organic group;
R^{E21} and R^{E22}: each independently a hydrogen atom or a monovalent organic group having 1-20 carbon atoms;
R⁶¹: a monovalent organic group having 1-20 carbon atoms;
R⁶²: a monovalent organic group having 1-20 carbon atoms;
E³¹: -CO- or -SO₂-.

7. A compound represented by the following formula (6): 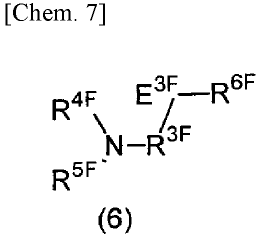 wherein definitions of substituents in the formula are as follows:
R^{3F}: a single bond or a group formed by fluorinating some or all of hydrogen atoms of a divalent organic group having 1-20 carbon atoms;
R^{4F}: a group formed by fluorinating some or all of hydrogen atoms of the following R⁴;
R^{5F}: a group formed by fluorinating some or all of hydrogen atoms of the following R⁵;
R⁴ and R⁵ are each independently a monovalent organic group having an electron-withdrawing group, a monovalent hydrocarbon group having 1-20 carbon atoms, or a monovalent hydrocarbon group having 2-20 carbon atoms which contains an etheric oxygen atom between carbon atoms, at least one of R⁴ and R⁵ being a monovalent organic group having an electron-withdrawing group, where R^{3F}, R^{4F}, and R^{5F} may be bonded to each other to form a divalent organic group or a trivalent organic group;
R^{6F}: a group formed by fluorinating some or all of hydrogen atoms of a monovalent organic group having 1-20 carbon atoms;
E^{3F}: a group formed by fluorinating some or all of hydrogen atoms of a divalent organic group formed by a reaction between the following E¹ and E²;
E¹: -COX, -SO₂X, or -C(R^{E11})(R^{E12})OH, where X is a hydroxyl group or a halogen atom, and R^{E11} and R^{E12} are each independently a hydrogen atom or a monovalent organic group having 1-10 carbon atoms;
E²: -C(R^{E21})(R^{E22})OH, -COX, or -SO₂X, where R^{E21} and R^{E22} are each independently a hydrogen atom or a monovalent organic group having 1-20 carbon atoms, and X is a hydroxyl group or a halogen atom.

8. A compound represented by the following formula (6a)-1, a compound represented by the following formula (6a)-2, a compound represented by the following formula (6b)-1, or a compound represented by the following formula (6b)-2: 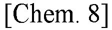 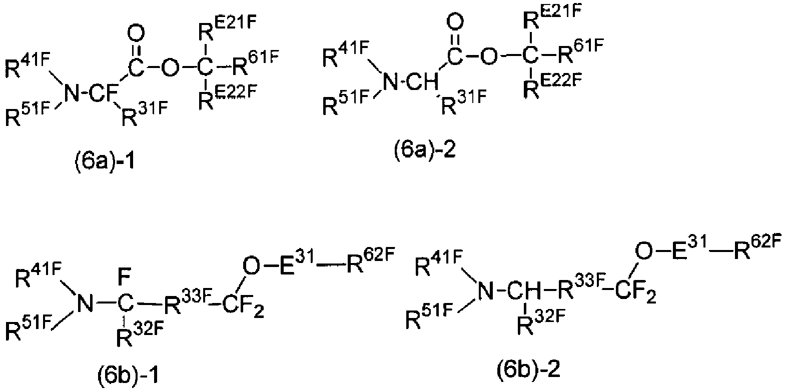 wherein definitions of substituents in the formulae are as follows:
R^{31F}: a fluorine atom or a group formed by fluorinating some or all of hydrogen atoms of a monovalent organic group having 1-19 carbon atoms;
R^{32F}: a fluorine atom or a group formed by fluorinating some or all of hydrogen atoms of a monovalent organic group having 1-19 carbon atoms;
R^{33F}: a single bond or a group formed by fluorinating some or all of hydrogen atoms of a divalent organic group having 1-10 carbon atoms;
R^{41F}: a group formed by fluorinating some or all of hydrogen atoms of the following R⁴¹;
R^{51F}: a group formed by fluorinating some or all of hydrogen atoms of the following R⁵¹;
R⁴¹: a monovalent organic group having an electron-withdrawing group, a monovalent hydrocarbon group having 1-20 carbon atoms, or a monovalent hydrocarbon group having 2-20 carbon atoms which contains an etheric oxygen atom between carbon atoms;
R⁵¹: a monovalent organic group having an electron-withdrawing group, where R^{31F}, R^{41F}, and R^{51F} may be bonded to each other to form a divalent organic group or a trivalent organic group, and R^{32F}, R^{41F}, and R^{51F} may be bonded to each other to form a divalent organic group or a trivalent organic group;
R^{E21F} and R^{E22F}: each independently a fluorine atom or a group formed by fluorinating some or all of the hydrogen atoms of a monovalent organic group having 1-20 carbon atoms;
R^{61F} and R^{62F}: each independently a group formed by fluorinating some or all of the hydrogen atoms of a monovalent organic group having 1-20 carbon atoms;
E³¹: -CO- or -SO₂-.

9. A method for producing a nitrogen-containing fluorine-containing compound, the method comprising a step in which a compound represented by the following formula (1a) is reacted with a compound having an amino-protecting group to obtain a compound represented by the following formula (3a)-1 and
a step in which the compound represented by the following formula (3a)-1 is reacted with a compound represented by the following formula (4a) to obtain a compound represented by the following formula (5a)
or comprising a step in which a compound represented by the following formula (1a) is reacted with a compound represented by the following formula (4a) to obtain a compound represented by the following formula (3a)-2 and
a step in which the compound represented by the following formula (3a)-2 is reacted with a compound having an amino-protecting group to obtain a compound represented by the following formula (5a),
the method further comprising a step in which the compound represented by the following formula (5a) is fluorinated to obtain at least one of a compound represented by the following formula (6a)-1 and a compound represented by the following formula (6a)-2: 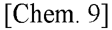 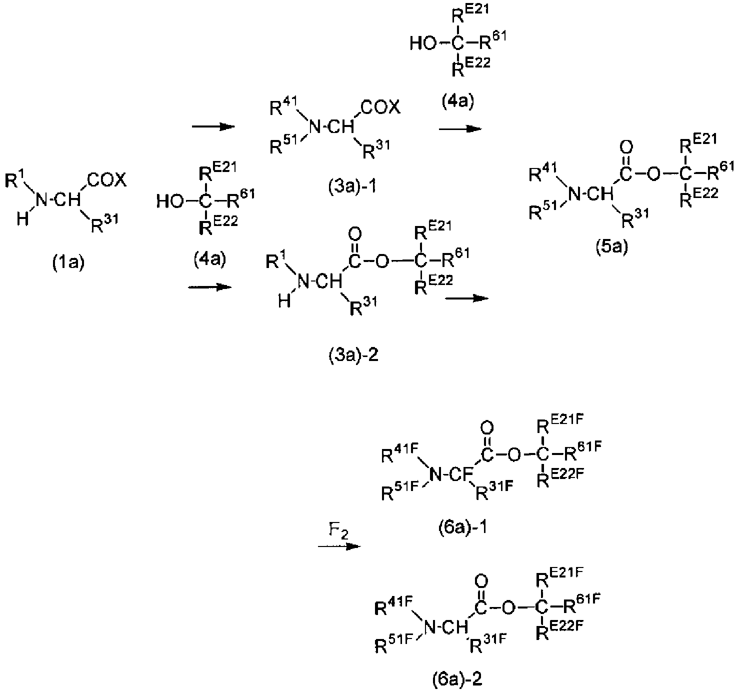
wherein definitions of substituents in the formulae are as follows:
R¹ is a hydrogen atom, a monovalent hydrocarbon group having 1-20 carbon atoms, or a monovalent hydrocarbon group having 2-20 carbon atoms which contains an etheric oxygen atom between carbon atoms, and in the case where R¹ is not a hydrogen atom, the R¹ may be bonded to R³¹ to form a divalent organic group;
R³¹: a hydrogen atom or a monovalent organic group having 1-19 carbon atoms;
X: a hydroxyl group or a halogen atom;
R⁴¹: a monovalent organic group having an electron-withdrawing group, a monovalent hydrocarbon group having 1-20 carbon atoms, or a monovalent hydrocarbon group having 2-20 carbon atoms which contains an etheric oxygen atom between carbon atoms;
R⁵¹: a monovalent organic group having an electron-withdrawing group, where R³¹, R⁴¹, and R⁵¹ may be bonded to each other to form a divalent organic group or a trivalent organic group;
R^{E21} and R^{E22}: each independently a hydrogen atom or a monovalent organic group having 1-20 carbon atoms;
R⁶¹: a monovalent organic group having 1-20 carbon atoms;
R^{41F}: a group formed by fluorinating some or all of hydrogen atoms of R⁴¹;
R^{51F}: a group formed by fluorinating some or all of hydrogen atoms of R⁵¹;
R^{61F}: a group formed by fluorinating some or all of hydrogen atoms of R⁶¹;
R^{E21F} and R^{E22F}: each independently a fluorine atom or a group formed by fluorinating some or all of the hydrogen atoms of a monovalent organic group having 1-20 carbon atoms.

10. A method for producing a nitrogen-containing fluorine-containing compound, the method comprising a step in which a compound represented by the following formula (1b) is reacted with a compound having an amino-protecting group to obtain a compound represented by the following formula (3b)-1 and
a step in which the compound represented by the following formula (3b)-1 is reacted with a compound represented by the following formula (4b) to obtain a compound represented by the following formula (5b)
or comprising a step in which a compound represented by the following formula (1b) is reacted with a compound represented by the following formula (4b) to obtain a compound represented by the following formula (3b)-2 and
a step in which the compound represented by the following formula (3b)-2 is reacted with a compound having an amino-protecting group to obtain a compound represented by the following formula (5b),
the method further comprising a step in which the compound represented by the following formula (5b) is fluorinated to obtain at least one of a compound represented by the following formula (6b)-1 and a compound represented by the following formula (6b)-2: 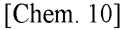 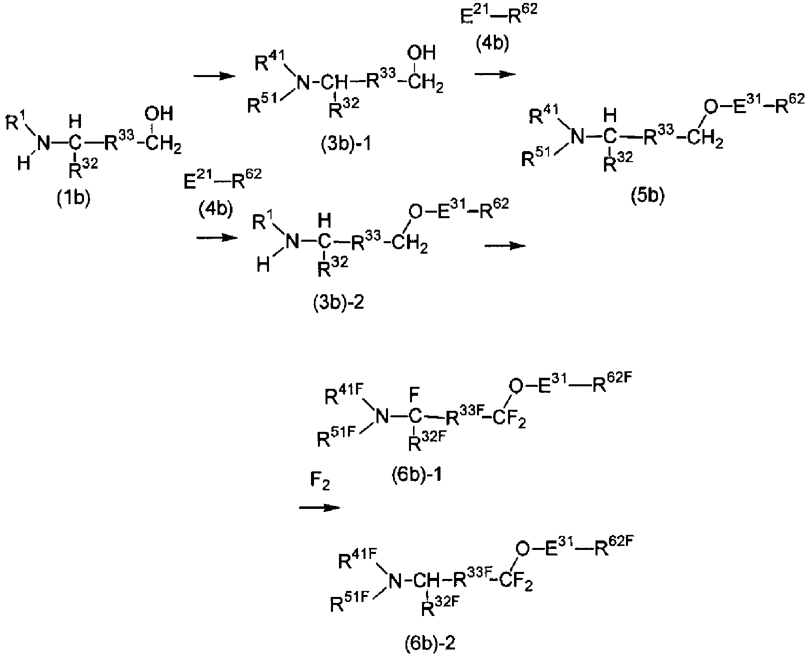
wherein definitions of substituents in the formulae are as follows:
R¹ is a hydrogen atom, a monovalent hydrocarbon group having 1-20 carbon atoms, or a monovalent hydrocarbon group having 2-20 carbon atoms which contains an etheric oxygen atom between carbon atoms, and in the case where R¹ is not a hydrogen atom, the R¹ may be bonded to R³² to form a divalent organic group;
R³²: a hydrogen atom or a monovalent organic group having 1-19 carbon atoms;
R³³: a single bond or a divalent organic group having 1-10 carbon atoms, a total number of carbon atoms of R³² and R³³ being 0-19;
R⁴¹: a monovalent organic group having an electron-withdrawing group, a monovalent hydrocarbon group having 1-20 carbon atoms, or a monovalent hydrocarbon group having 2-20 carbon atoms which contains an etheric oxygen atom between carbon atoms;
R⁵¹: a monovalent organic group having an electron-withdrawing group, where R³², R⁴¹, and R⁵¹ may be bonded to each other to form a divalent organic group or a trivalent organic group;
E²¹: -COX or -SO₂X, where X is a hydroxyl group or a halogen atom;
R⁶²: a monovalent organic group having 1-20 carbon atoms;
E³¹: -CO- or -SO₂-;
R^{32F}: a group formed by fluorinating some or all of hydrogen atoms of R³²;
R^{33F}: a group formed by fluorinating some or all of hydrogen atoms of R³³, a total number of carbon atoms of R^{32F} and R^{33F} being 0-19;
R^{41F}: a group formed by fluorinating some or all of hydrogen atoms of R⁴¹;
R^{51F}: a group formed by fluorinating some or all of hydrogen atoms of R⁵¹;
R^{62F}: a group formed by fluorinating some or all of hydrogen atoms of R⁶².
